(19) Europäisches Patentamt
European Patent Office
Office européen des brevets

(11) EP 4 702 990 A1

(12) **EUROPEAN PATENT APPLICATION**
published in accordance with Art. 153(4) EPC

(43) Date of publication:
**04.03.2026 Bulletin 2026/10**

(21) Application number: **24796043.8**

(22) Date of filing: **23.04.2024**

(51) International Patent Classification (IPC):
***A61K 47/68*** *(2017.01)* ***A61K 31/4745*** *(2006.01)*
***A61P 35/00*** *(2006.01)*

(52) Cooperative Patent Classification (CPC):
**A61K 31/4745; A61K 47/68; A61P 35/00**

(86) International application number:
**PCT/CN2024/089266**

(87) International publication number:
**WO 2024/222662 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.04.2023 CN 202310441120**

(71) Applicant: **CSPC Megalith Biopharmaceutical Co., Ltd.**
**Shijiazhuang, Hebei 050025 (CN)**

(72) Inventors:
- **ZHANG, Liqiong**
  **Shijiazhuang, Hebei 050025 (CN)**
- **WANG, Juan**
  **Shijiazhuang, Hebei 050025 (CN)**
- **WAN, Xuechao**
  **Shijiazhuang, Hebei 050025 (CN)**
- **DAN, Mo**
  **Shijiazhuang, Hebei 050025 (CN)**
- **HUI, Xiwu**
  **Shijiazhuang, Hebei 050025 (CN)**
- **LIU, Shengnan**
  **Shijiazhuang, Hebei 050025 (CN)**
- **QI, Huanhuan**
  **Shijiazhuang, Hebei 050025 (CN)**
- **JIA, Ranran**
  **Shijiazhuang, Hebei 050025 (CN)**
- **ZHANG, Lixuan**
  **Shijiazhuang, Hebei 050025 (CN)**
- **GE, Lan**
  **Shijiazhuang, Hebei 050025 (CN)**

(74) Representative: **Barrow, Nicholas Martin et al**
**Venner Shipley LLP**
**406 Cambridge Science Park**
**Milton Road**
**Cambridge CB4 0WW (GB)**

# (54) USE OF ANTIBODY DRUG CONJUGATE

(57) A pharmaceutical composition of an antibody-drug conjugate, or a pharmaceutically acceptable salt, hydrate, solvate, stereoisomer, or isotopic label thereof, the use thereof in treating a tumor, and a method therewith for treating a tumor.

EP 4 702 990 A1

## Description

### Technical Field

This application belongs to the field of biomedicine and specifically relates to the use of an antibody-drug conjugate.

### Background of Art

The epidermal growth factor receptor (EGFR) is a member of the HER family. EGFR is a 170-kDa transmembrane glycoprotein composed of 1186 amino acids. It consists of three parts: an extracellular receptor domain, a transmembrane domain, and an intracellular tyrosine kinase domain. Ligands that have been confirmed to be able to bind to EGFR include: epidermal growth factor (EGF), transforming growth factor alpha (TGF$\alpha$), amphiregulin, heparin-binding EGF, epiregulin, etc. In human tissues, EGF and TGF$\alpha$ are considered the two most important ligands for EGFR.

EGFR is a constitutively expressed component in many normal epithelial tissues, such as the skin and hair follicles. Additionally, EGFR is highly expressed in various human malignant tumor tissues: lung cancer (40%-80%), breast cancer (14%-91%), gastric cancer (33%-74%), colon cancer (25%-77%), pancreatic cancer (30%-50%), prostate cancer (40%-80%), renal cancer (50%-90%), ovarian cancer (35%-70%), and head and neck cancer (36%-100%). EGFR is overexpressed and/or mutated in most tumors and is associated with poor prognosis. In vitro studies and experimental animal model investigations indicate that EGFR activation is involved in regulating several critical cellular functions in cell transformation and tumor cell progression: proliferation/differentiation, survival, induction of angiogenesis, metastatic potential, and resistance to chemotherapy and radiotherapy (Normanno et al., 2003). Therefore, EGFR has become a hotspot for antitumor drug development.

Multiple studies have confirmed that EGFR-targeted inhibitors exhibit antitumor activity, and several drugs have been approved for marketing in China and other countries. These include small-molecule tyrosine kinase inhibitors (e.g., gefitinib, afatinib, osimertinib, etc), monoclonal antibodies (e.g., cetuximab, nimotuzumab, etc), bispecific antibodies (e.g., amivantamab), and antibody-drug conjugates (e.g., cetuximab sarotalocan). Indications include non-small cell lung cancer (NSCLC), head and neck squamous carcinoma, colorectal cancer, nasopharyngeal carcinoma, and the like, having EGFR-positive expression and/or mutation. Cetuximab and other EGFR monoclonal antibodies have also been extensively explored in other tumors, such as triple-negative breast cancer, gastric cancer, pancreatic cancer, and the like, suggesting their antitumor effects in these cancer types.

Antibody-drug conjugates (ADCs) targeting EGFR have emerged as a hotspot in drug development to overcome resistance to epidermal growth factor receptor tyrosine kinase inhibitors (EGFR-TKIs) and EGFR monoclonal antibodies. Currently approved EGFR-ADC products include Akalux (cetuximab sarotalocan) by Rakuten Medical, which received accelerated approval in Japan on September 25, 2020, for treating unresectable locally advanced or recurrent head and neck cancer. It is the world's first approved photoimmunotherapy drug. Domestically, MRG003 by MeiYaKe has progressed rapidly and entered Phase II clinical trial.

CN202211461614.0 discloses an EGFR-targeted antibody-drug conjugate:

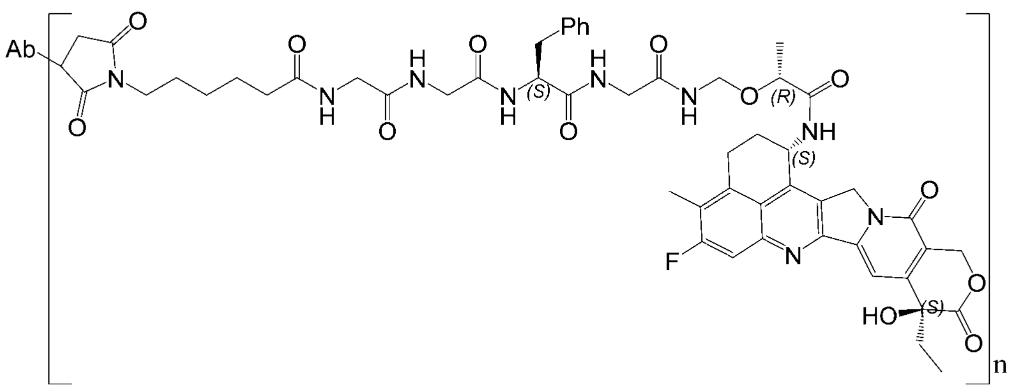

wherein Ab is an EGFR-targeted antibody.

In vitro studies show that this ADC effectively inhibits various tumor cells. However, the therapeutic efficacy of this ADC against malignant tumors, especially advanced malignant tumors such as advanced solid tumors, requires further investigation.

## Summary of the Invention

The present invention provides a use of an antibody-drug conjugate.

In one aspect, the present invention provides an antibody-drug conjugate represented by Formula I, or a pharmaceutically acceptable salt, hydrate, solvate, stereoisomer, or isotopic label thereof:

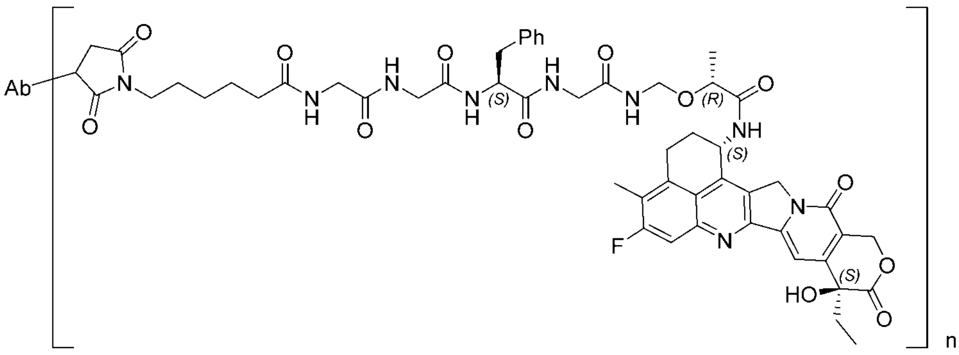

(I)

wherein Ab is selected from SWY2110, SWY2111, SWY2112, and SWY2113;

SWY2110 is an antibody having two heavy chains with the amino acid sequence shown in SEQ ID NO:29 and two light chains with the amino acid sequence shown in SEQ ID NO:30;
SWY2111 is an antibody having two heavy chains with the amino acid sequence shown in SEQ ID NO:34 and two light chains with the amino acid sequence shown in SEQ ID NO:30;
SWY2112 is an antibody having two heavy chains with the amino acid sequence shown in SEQ ID NO:37 and two light chains with the amino acid sequence shown in SEQ ID NO:30;
SWY2113 is an antibody having two heavy chains with the amino acid sequence shown in SEQ ID NO:40 and two light chains with the amino acid sequence shown in SEQ ID NO:30;
n is an integer of 1-8 or a decimal of 1-8.

Further, n is an integer of 4-8 or a decimal of 4-8.

Further, when n is an integer of 1-8, it can be 1, 2, 3, 4, 5, 6, 7, or 8; when n is an integer of 4-8, it can be 4, 5, 6, 7, or 8.

Further, the antibody-drug conjugate represented by Formula I has structures shown in Formulae I-1 to I-4:

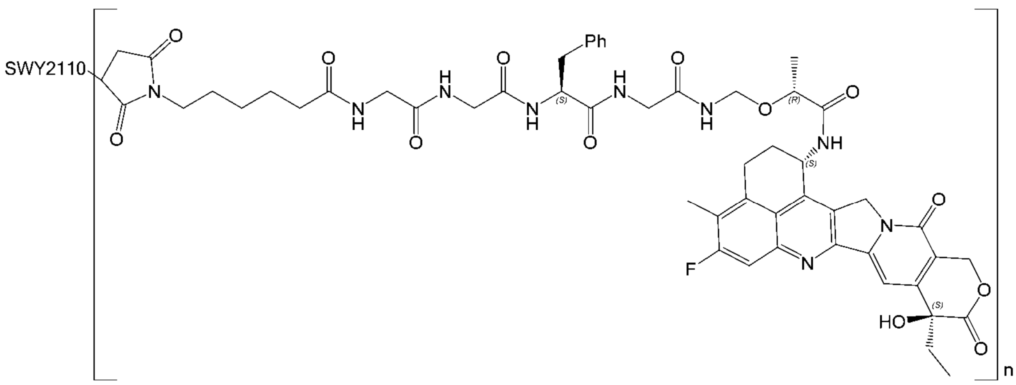

I-1

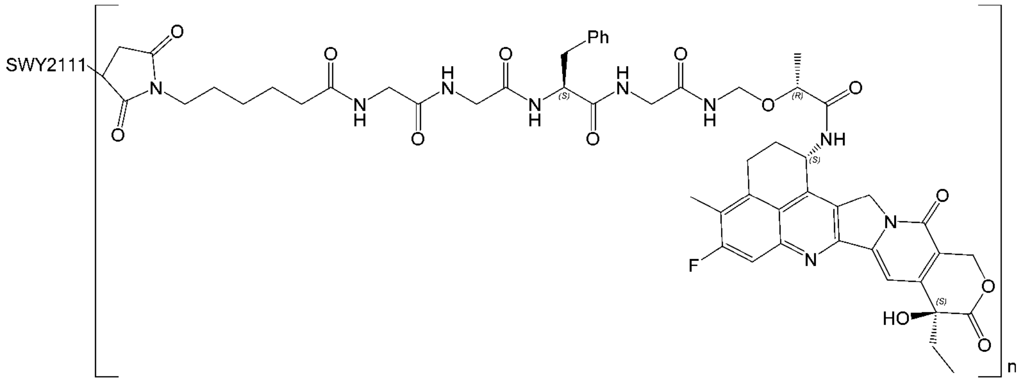

I-2

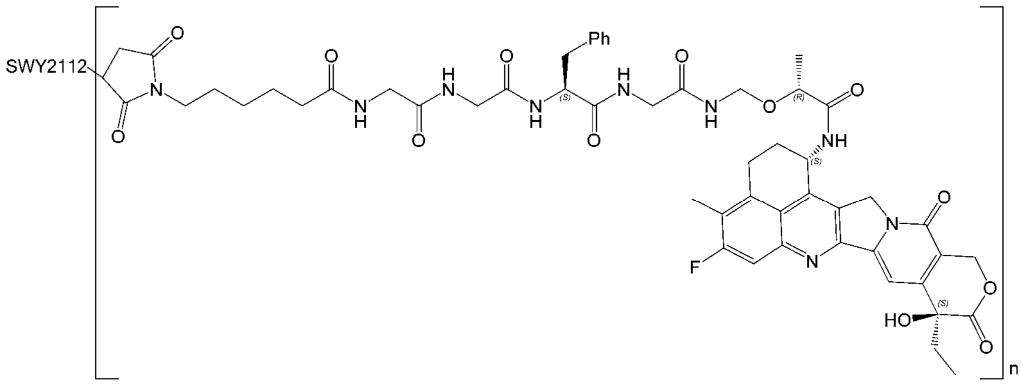

I-3

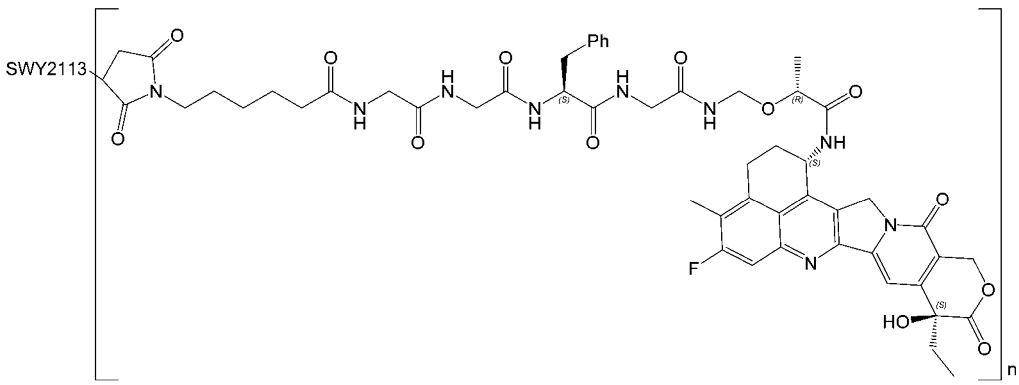

I-4

The present invention provides the use of an antibody-drug conjugate represented by Formula I, or a pharmaceutically acceptable salt, hydrate, solvate, stereoisomer, or isotopic label thereof in manufacture of a medicament for treating a tumor, which is selected from colorectal cancer, triple-negative breast cancer, gastric cancer, esophageal cancer, biliary tract cancer, HR-positive breast cancer, liver cancer, ovarian cancer, endometrial cancer, prostate cancer, urothelial carcinoma, lung squamous carcinoma, pancreatic cancer, cervical cancer, non-squamous non-small cell lung cancer, head and neck squamous carcinoma (including nasopharyngeal carcinoma), kidney cancer, and the like.

Further, the tumor is selected from EGFR moderate/high-expression triple-negative breast cancer, EGFR moderate/high-expression gastric cancer, esophageal cancer, pancreatic cancer, cervical cancer, biliary tract cancer, non-squamous non-small cell lung cancer, HR-positive breast cancer, liver cancer, ovarian cancer, endometrial cancer, prostate cancer, urothelial carcinoma, kidney cancer etc, and RAS/BRAF mutant colorectal cancer, EGFR moderate/high-expression lung squamous carcinoma (Sq-NSCLC), EGFR mutant non-squamous non-small cell lung cancer (Nsq-NSCLC), EGFR moderate/high-expression head and neck squamous carcinoma (including nasopharyngeal carcinoma), RAS/BRAF wild type EGFR moderate/high-expression colorectal cancer, and the like.

The present invention provides the use of an antibody-drug conjugate represented by Formula I, or a pharmaceutically acceptable salt, hydrate, solvate, stereoisomer, or isotopic label thereof in preventing and treating a tumor, which is selected from triple-negative breast cancer, gastric cancer, esophageal cancer, pancreatic cancer, cervical cancer, biliary tract cancer, HR-positive breast cancer, liver cancer, ovarian cancer, endometrial cancer, prostate cancer, urothelial carcinoma, lung squamous carcinoma, non-squamous non-small cell lung cancer, head and neck squamous carcinoma

(including nasopharyngeal carcinoma), colorectal cancer, kidney cancer, and the like.

Further, the tumor is selected from EGFR moderate/high-expression triple-negative breast cancer, EGFR moderate/high-expression gastric cancer, esophageal cancer, pancreatic cancer, cervical cancer, biliary tract cancer, non-squamous non-small cell lung cancer, HR-positive breast cancer, liver cancer, ovarian cancer, endometrial cancer, prostate cancer, urothelial carcinoma, kidney cancer, EGFR moderate/high-expression lung squamous carcinoma (Sq-NSCLC), EGFR mutant non-squamous non-small cell lung cancer (Nsq-NSCLC), EGFR moderate/high-expression head and neck squamous carcinoma (including nasopharyngeal carcinoma), RAS/BRAF wild type EGFR moderate/high-expression colorectal cancer etc, and RAS/BRAF mutant colorectal cancer, and the like.

The antibody-drug conjugate represented by Formula I, or a pharmaceutically acceptable salt, hydrate, solvate, stereoisomer, or isotopic label thereof, is administered to a patient in need of treatment by clinically acceptable routes, such as oral administration or injection (e.g., intravenous injection, intramuscular injection), preferably intravenous injection.

The antibody-drug conjugate represented by Formula I, or a pharmaceutically acceptable salt, hydrate, solvate, stereoisomer, or isotopic label thereof, is formulated into clinically acceptable pharmaceutical preparations, such as oral preparations or injection preparations. The oral preparations include tablets and capsules; the injection preparations include injection solutions and lyophilised powder for injection. Optionally, the pharmaceutical preparations contain pharmaceutically acceptable excipients. Each dosing unit of the pharmaceutical preparation contains the antibody-drug conjugate represented by Formula I, or a pharmaceutically acceptable salt, hydrate, solvate, stereoisomer, or isotopic label thereof, calculated as the antibody-drug conjugate represented by Formula I, in an amount of 1-1000mg, preferably 5-500mg, or 10-300mg, or 20-200mg, or 50-150mg, or 50-300mg, or 50-400mg, or 100-200mg, or 100-300mg, or 100-500mg, more preferably 1mg, 5mg, 10mg, 20mg, 30mg, 40mg, 50mg, 60mg, 70mg, 80mg, 90mg, 100mg, 150mg, 200mg, 250mg, 300mg, 500mg, 1000mg, or the like. The pharmaceutical preparation can be administered as a single dose or in divided doses.

The antibody-drug conjugate represented by Formula I, or a pharmaceutically acceptable salt, hydrate, solvate, stereoisomer, or isotopic label thereof, is administered at a therapeutically effective amount to a subject or patient in need of treatment to treat tumors. The "therapeutically effective amount" refers to a dose sufficient to alleviate or inhibit the tumor growth, or eliminate the tumor, benefiting the subject or patient, without causing intolerable toxic side effects.

In some embodiments, the therapeutically effective amount per administration (calculated as the antibody-drug conjugate represented by Formula I) is 1-1000 mg/dosing, preferably 5-500 mg/dosing, or 10-300 mg/dosing, or 20-200 mg/dosing, or 50-150 mg/dosing, or 50-300 mg/dosing, or 50-400 mg/dosing, or 100-200 mg/dosing, or 100-300 mg/dosing, or 100-500 mg/dosing, more preferably 5 mg/dosing, 6 mg/dosing, 8 mg/dosing, 10 mg/dosing, 15 mg/dosing, 20 mg/dosing, 25 mg/dosing, 30 mg/dosing, 40 mg/dosing, 50 mg/dosing, 60 mg/dosing, 70 mg/dosing, 80 mg/dosing, 90 mg/dosing, 100 mg/dosing, 150 mg/dosing, 200 mg/dosing, 250 mg/dosing, 300 mg/dosing, 500 mg/dosing, 600 mg/dosing, 700 mg/dosing, 800 mg/dosing, 900 mg/dosing, or the like.

In some embodiments, the therapeutically effective amount (calculated as the antibody-drug conjugate represented by Formula I) is 1mg/day to 1000 mg/day, preferably 5mg-500 mg/day, or 10-300 mg/day, or 20-200 mg/day, or 50-150 mg/day, or 50-300 mg/day, or 50-400 mg/day, or 100-200 mg/day, or 100-300 mg/day, or 100-500 mg/day, more preferably 10 mg/day, 20 mg/day, 30 mg/day, 40 mg/day, 50 mg/day, 60 mg/day, 70 mg/day, 80 mg/day, 90 mg/day, 100 mg/day, 150 mg/day, 200 mg/day, 250 mg/day, 300 mg/day, 500 mg/day, 600 mg/day, 700 mg/day, 800 mg/day, 900mg/day, or the like.

In some embodiments, the therapeutically effective amount (calculated as the antibody-drug conjugate represented by Formula I and the body weight of patient, wherein the amount in mg refers to the dose of the antibody-drug conjugate represented by Formula I, and the amount in kg refers to the weight of patient body) is 0.05-50 mg/kg, preferably 0.1-20 mg/kg, or 0.2-20 mg/kg, or 0.5-20 mg/kg, or 0.5-15 mg/kg, or 0.5-10 mg/kg, or 0.5-8 mg/kg, or 0.5-6 mg/kg, or 0.5-4 mg/kg, or 0.5-2 mg/kg, or 1-20 mg/kg, or 1-10 mg/kg, or 1-8 mg/kg, or 2-20 mg/kg, or 2-10 mg/kg, or 2-8 mg/kg, or 3-20 mg/kg, or 3-10 mg/kg, or 3-8 mg/kg, or 5-20 mg/kg, or 5-10 mg/kg, or 0.3 mg/kg, or 0.4 mg/kg, or 0.6 mg/kg, or 0.8 mg/kg, or 1.5 mg/kg, or 1.8 mg/kg, or 2.0 mg/kg, or 2.2 mg/kg, or 2.4 mg/kg, or 2.5 mg/kg, or 2.8 mg/kg, or 3 mg/kg, or 3.2 mg/kg, or 3.5 mg/kg, or 3.6 mg/kg, or 3.8 mg/kg, or 4.0 mg/kg, or 4.2 mg/kg, or 4.4 mg/kg, or 4.5 mg/kg, or 4.6 mg/kg, or 4.8 mg/kg, or 5.0 mg/kg, or 5.2 mg/kg, or 5.4 mg/kg, or 5.5 mg/kg, or 5.6 mg/kg, or 5.8 mg/kg, or 6.0 mg/kg, or 6.2 mg/kg, or 6.4 mg/kg, or 6.8 mg/kg, or 7.0 mg/kg, or 7.2 mg/kg, or 7.5 mg/kg, or 7.8 mg/kg, or 8.0 mg/kg, or 8.5 mg/kg, or 9.0 mg/kg, or 9.5 mg/kg, or 11 mg/kg, or 11.5 mg/kg, or 12.0 mg/kg, or 16.8 mg/kg, or 18.0mg/kg.

The above therapeutically effective amount can be administered once daily (QD) or can be administered by dividing the therapeutically effective amount into multiple administrations within one day, for example, twice daily (BID) or three times daily (TID). The above therapeutically effective amount can be administered once weekly (QW) or can be administered by dividing the aforementioned therapeutically effective amount as the total weekly dosage into multiple-day administrations within a week, for example, twice weekly (BIW) or thrice weekly (TIW). The above therapeutically effective amount can be administered once every two weeks (Q2W), or the above therapeutically effective amount can be administered as the total two-week dosage by dividing into multiple-day administrations within two weeks, for example,

twice every two weeks (BI2W) or three times every two weeks (TI2W). The above therapeutically effective amount can be administered once every three weeks (Q3W), or the above therapeutically effective amount can be administered as the total three-week dosage by dividing into multiple-day administrations within three weeks, for example, twice every three weeks (BI3W, e.g. Day 1 (D1) and Day 8 (D8) in one cycle every three weeks) or three times every three weeks (TI3W). The above therapeutically effective amount can be administered once monthly (QM), or the above therapeutically effective amount can be administered as the total monthly dosage by dividing into multiple-day administrations within one month, for example, twice monthly (BIM) or three times monthly (TIM). The above therapeutically effective amount can also be administered at intervals e.g. administrated once at an interval of 2-7 days, or administrated once at an interval of 3 days, or 4 days, or 5 days, or 6 days, or administrated once at an interval of 2-4 weeks, e.g. administrated once at an interval of 2 weeks (administrated once every 3 weeks with a cycle of every 3 weeks), administrated once at an interval of 1 week (once every 2 weeks with a cycle of every 2 weeks or a cycle of every 4 weeks) and administrated once at an interval of 3 weeks (once every 4 weeks with a cycle of every 4 weeks), or administrated once at an interval of 1-3 months, e.g. administrated once at an interval of 1 month (every 2 months); or continuously administered for 3 weeks and then stopped for 1 week with a cycle of every 4 weeks, or continuously administered for 2 weeks and then stopped for 1 week with a cycle of every 3 weeks, or continuously administered for 2 weeks and then stopped for 2 weeks with a cycle of every 4 weeks, or administered at an interval of 1 week (once every 2 weeks) with a cycle of every 4 weeks. An exemplary administration method includes: taking every 3 weeks as a cycle and administering once on Day 1 in each cycle.

For conciseness in description, the term "about" is not used for some quantitative data herein. It should be understood that, whether the term "about" is explicitly used or not, each numerical value given here includes not only the actual given value (an given value), but also an approximation of this given value reasonably inferred by those skilled in the art, including an equivalent to or an approximation of this given value produced from experimental and/or measurement conditions. The approximation is preferably in the range of ±20%, ±15%, ±10%, ±8%, ±6%, ±5%, ±4%, ±3%, ±2%, or ±1% of the given value.

Clinical studies have shown that the antibody-drug conjugate represented by Formula I, or a pharmaceutically acceptable salt, hydrate, solvate, stereoisomer, or isotopic label thereof can effectively treat advanced malignant tumors, especially EGFR-positive advanced solid tumors, can overcome the resistance of EGFR-TKIs and EGFR monoclonal antibodies, and provide superior clinical benefits compared to existing treatments for patients with advanced solid tumors including non-small cell lung cancer, and the state of disease can be controlled or alleviated.

Antibody Sequence Description

1. EGFR antibody SWY2110

Light chain (SEQ ID NO:30) :

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

Heavy chain (SEQ ID NO:29) :

QVQLQESGPGLVKPSETLSLTCTVSGFSLSNYDVHWVRQAPGKGLEWLGVIWSGGNTDYNTPF
TSRLTISVDTSKNQFSLKLSSVTAADTAVYYCARALDYYDYEFAYWGQGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLN

GKEYKCKVSNKALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEALHNHYTQKSLS LSPGK

SWY2110 HCDR1: NYDVH SEQ ID NO:21
SWY2110 HCDR2: VIWSGGNTDYNTPFTS SEQ ID NO:22
SWY2110 HCDR3: ALDYYDYEFAY SEQ ID NO:23
SWY2110 LCDR1: RASQSIGTNIH SEQ ID NO:24
SWY2110 LCDR2: YASESIS SEQ ID NO:25
SWY2110 LCDR3: QQNNEWPTS SEQ ID NO:26
SWY2110 HV: SEQ ID NO:27

QVQLQESGPGLVKPSETLSLTCTVSGFSLSNYDVHWVRQAPGKGLEWLGVIWSGGNTDYNTPF TSRLTISVDTSKNQFSLKLSSVTAADTAVYYCARALDYYDYEFAYWGQGTLVTVSS

SWY2110 LV: SEQ ID NO:28

EIVLTQSPDFQSVTPKEKVTITCRASQSIGTNIHWYQQKPDQSPKLLIKYASESISGIPSRFSGSGS GTDFTLTINSLEAEDAATYYCQQNNEWPTSFGQGTKLEIK

2. EGFR antibody SWY2111

Light chain (SEQ ID NO:30) :

EIVLTQSPDFQSVTPKEKVTITCRASQSIGTNIHWYQQKPDQSPKLLIKYASESISGIPSRFSGSGS GTDFTLTINSLEAEDAATYYCQQNNEWPTSFGQGTKLEIK RTVAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGEC

Heavy chain (SEQ ID NO:34) :

QVQLQESGPGLVKPSETLSLTCTVSGFSLSDYDVHWVRQAPGKGLEWLGVIWSGGNTDYNTPF TSRLTISVDTSKNQFSLKLSSVTAADTAVYYCARALDDYDYEFAYWGQGTLVTVSSASTKGPS VFPLAPSSKSTSGGTAALGCLVKDYFPEPVTVSWNSGALTSGVHTFPAVLQSSGLYSLSSVVTV PSSSLGTQTYICNVNHKPSNTKVDKRVEPKSCDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEALHNHYTQKSLS LSPGK

SWY2111 HCDR1: DYDVH SEQ ID NO:31
SWY2111 HCDR2: VIWSGGNTDYNTPFTS SEQ ID NO:22
SWY2111 HCDR3: ALDDYDYEFAY SEQ ID NO:32
SWY2111 LCDR1: RASQSIGTNIH SEQ ID NO:24
SWY2111 LCDR2: YASESIS SEQ ID NO:25
SWY2111 LCDR3: QQNNEWPTS SEQ ID NO:26
SWY2111 HV: SEQ ID NO:33

QVQLQESGPGLVKPSETLSLTCTVSGFSLSDYDVHWVRQAPGKGLEWLGVIWSGGNTDYNTPF
TSRLTISVDTSKNQFSLKLSSVTAADTAVYYCARALDDYDYEFAYWGQGTLVTVSS

SWY2111 LV: SEQ ID NO:28

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK

3. EGFR antibody SWY2112

Light chain (SEQ ID NO:30) :

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

Heavy chain (SEQ ID NO:37) :

QVQLQESGPGLVKPSETLSLTCTVSGFSLSEYDVHWVRQAPGKGLEWLGVIWSGGNTDYNTPF
TSRLTISVDTSKNQFSLKLSSVTAADTAVYYCARALDDYDYEFAYWGQGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYTQKSLS
LSPGK

SWY2112 HCDR1: EYDVH SEQ ID NO:35
SWY2112 HCDR2: VIWSGGNTDYNTPFTS SEQ ID NO:22
SWY2112 HCDR3: ALDDYDYEFAY SEQ ID NO:32
SWY2112 LCDR1: RASQSIGTNIH SEQ ID NO:24
SWY2112 LCDR2: YASESIS SEQ ID NO:25
SWY2112 LCDR3: QQNNEWPTS SEQ ID NO:26
SWY2112 HV: SEQ ID NO:36

QVQLQESGPGLVKPSETLSLTCTVSGFSLSEYDVHWVRQAPGKGLEWLGVIWSGGNTDYNTPF
TSRLTISVDTSKNQFSLKLSSVTAADTAVYYCARALDDYDYEFAYWGQGTLVTVSS

SWY2112 LV: SEQ ID NO:28

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK

4. EGFR antibody SWY2113

Light chain (SEQ ID NO:30) :

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

Heavy chain (SEQ ID NO:40) :

QVQLQESGPGLVKPSETLSLTCTVSGFSLSHYDVHWVRQAPGKGLEWLGVIWSGGNTDYNTPF
TSRLTISVDTSKNQFSLKLSSVTAADTAVYYCARALDDYDYEFAYWGQGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYTQKSLS
LSPGK

SWY2113 HCDR1: HYDVH SEQ ID NO:38
SWY2113 HCDR2: VIWSGGNTDYNTPFTS SEQ ID NO:22
SWY2113 HCDR3: ALDDYDYEFAY SEQ ID NO:32
SWY2113 LCDR1: RASQSIGTNIH SEQ ID NO:24
SWY2113 LCDR2: YASESIS SEQ ID NO:25
SWY2113 LCDR3: QQNNEWPTS SEQ ID NO:26
SWY2113 HV: SEQ ID NO:39

QVQLQESGPGLVKPSETLSLTCTVSGFSLSHYDVHWVRQAPGKGLEWLGVIWSGGNTDYNTPF
TSRLTISVDTSKNQFSLKLSSVTAADTAVYYCARALDDYDYEFAYWGQGTLVTVSS

SWY2113 LV: SEQ ID NO:28

EIVLTQSPDFQSVTPKEKVTITCRASQSIGTNIHWYQQKPDQSPKLLIKYASESISGIPSRFSGSGS
GTDFTLTINSLEAEDAATYYCQQNNEWPTSFGQGTKLEIK

## Description of the drawings

Figure 1 shows the detection result for the DAR value of SWY2110-JSSW-001.
Figure 2 shows the detection result for the DAR value of SWY2111-JSSW-001.
Figure 3 shows the detection result for the DAR value of SWY2112-JSSW-001.
Figure 4 shows the detection result for the DAR value of SWY2113-JSSW-001.
Figure 5 shows the detection result for the DAR value of SWY2110-Dxd.
Figure 6 shows the detection result for the DAR value of SWY2110-Vc MMAE.
Figure 7 shows the effect of SWY2110-ADC on the tumor volume of human colorectal cancer DiFi xenograft tumor in NU/NU mice.

Figure 8 shows the effect of SWY2110-ADC on the tumor volume of human lung cancer PC9-GR (gefitinib-resistant cell) xenograft tumor in NU/NU mice.
Figure 9 shows the detection result of the inhibitory activity of SWY2110-ADC on DiFi cells.
Figure 10 shows the detection result of the inhibitory activity of SWY2110-ADC on PC9-GR cells.
Figure 11 shows the effect of SWY2110-ADC on the tumor volume of human lung cancer PC9-AR (PC9-Del19/T790M/C797S, osimertinib-resistant cell) xenograft tumor in NU/NU mice.
Figure 12 shows the effect of SWY2110-ADC, SWY2111-ADC, SWY2112-ADC, and SWY2113-ADC on the tumor volume of human lung cancer NCI-H1975 xenograft tumor in NU/NU mice.
Figure 13 shows the effect of SWY2110-ADC, SWY2111-ADC, SWY2112-ADC, and SWY2113-ADC on the tumor volume of human breast cancer MDA-MB-468 xenograft tumor in NOD-SCID mice.
Figure 14 shows the effect of SWY2110-ADC, SWY2111-ADC, SWY2112-ADC, and SWY2113-ADC on the tumor volume of human colorectal cancer DiFi xenograft tumor in NU/NU mice.
Figure 15 shows the effect of SWY2110-ADC, SWY2111-ADC, SWY2112-ADC, and SWY2113-ADC on the tumor volume of human lung cancer PC9-GR (gefitinib-resistant cell) xenograft tumor in NU/NU mice.

## Detailed description

The present invention is further described below in connection with specific examples. It should be understood that these examples are intended to illustrate the present invention only and are not intended to limit the scope of the present invention. Experimental methods for which no specific conditions are indicated in the following examples are generally in accordance with conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of the present invention. The preferred embodiments and materials shown in this disclosure are illustrative only.

The structures of the compounds according to the present invention are determined by nuclear magnetic resonance (NMR) and/or liquid chromatograph mass spectrometry (LC-MS) and/or liquid chromatography (HPLC). The instrument used for NMR is a Bruker Avance III 400 MHz nuclear magnetic resonance apparatus; the instrument used for LC-MS is a SHIMADZU LC-20AD-PDA-LCMS-2020; and the instrument used for HPLC is a SHIMADZU LC-20AD-PDA high-performance liquid chromatograph. The starting materials in the examples of the present invention are known and commercially available, or can be synthesized using those methods known in the art or according to those methods known in the art. The antibodies of the present invention can be prepared by hybridoma technology described by Kohler et al., Nature (1975), for the first time, or by recombinant DNA method (U.S. Patent 4,816,567, etc.).

Table 1: Abbreviations

| Abbreviations and Terms | Full Name in English |
|---|---|
| ADA | Anti-Drug Antibody |
| ADR | Adverse Drug Reaction |
| AE | Adverse Event |
| AUC | Area Under Curve |
| CL | Clearance |
| Cmax | Maximum Concentration |
| DCR | Disease Control Rate |
| DLT | Dose Limited Toxicity |
| DoR | Duration of Response |
| ECOG | Eastern Cooperative Oncology Group |
| EGFR | Epidermal Growth Factor Receptor |
| EGFR-TKI | Epidermal Growth Factor Receptor Tyrosine Kinase Inhibitor |
| EOT | End of Treatment |
| HNSTD | Highest Non-Severely Toxic Dose |
| MTD | Maximum Tolerated Dose |
| MRT | Mean Residence Time |
| NCI-CTCAE | National Cancer Institute<br>Common Terminology Criteria for Adverse Events |
| NSCLC | Non-small Cell Lung Cancer |

(continued)

| Abbreviations and Terms | Full Name in English |
|---|---|
| Nsq-NSCLC | Non-squamous Non-small Cell Lung Cancer |
| ORR | Objective Response Rate |
| OS | Overall Survival |
| PFS | Progression-Free-Survival |
| PK | Pharmacokinetics |
| PT | Prothrombin Time |
| Q3W | Once every three weeks |
| QW | Once weekly |
| SAE | Serious Adverse Event |
| Sq-NSCLC | Squamous Non-small Cell Lung Cancer |
| SUSAR | Suspected Unexpected Serious Adverse Reaction |
| TEAE | Treatment Emergent Adverse Event |
| Tmax | Time to Maximum Concentration |
| TRAE | Treatment-related Adverse Events |

Preparation Examples

Example 1: Preparation of linker-drug compound

N-((2R,10S)-10-benzyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-methyl-1,6,9,12,15-pentaoxo-3-oxa-5,8,11,14-tetraazahexadecan-16-yl)-6-(2,5-dioxo-2,5-dihydro-1H-pyrrol-1-yl)hexanamide (JSSW-001)

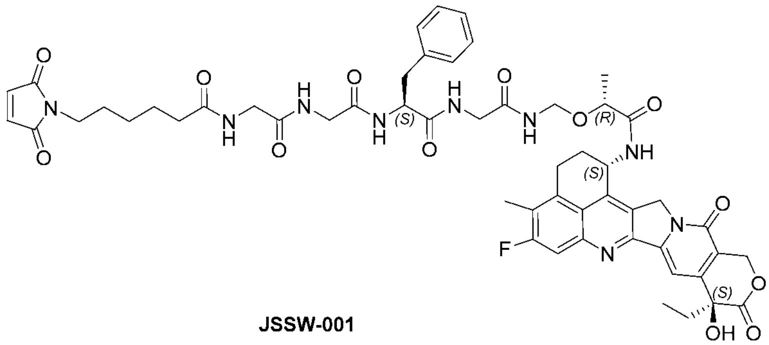

**JSSW-001**

Scheme I:

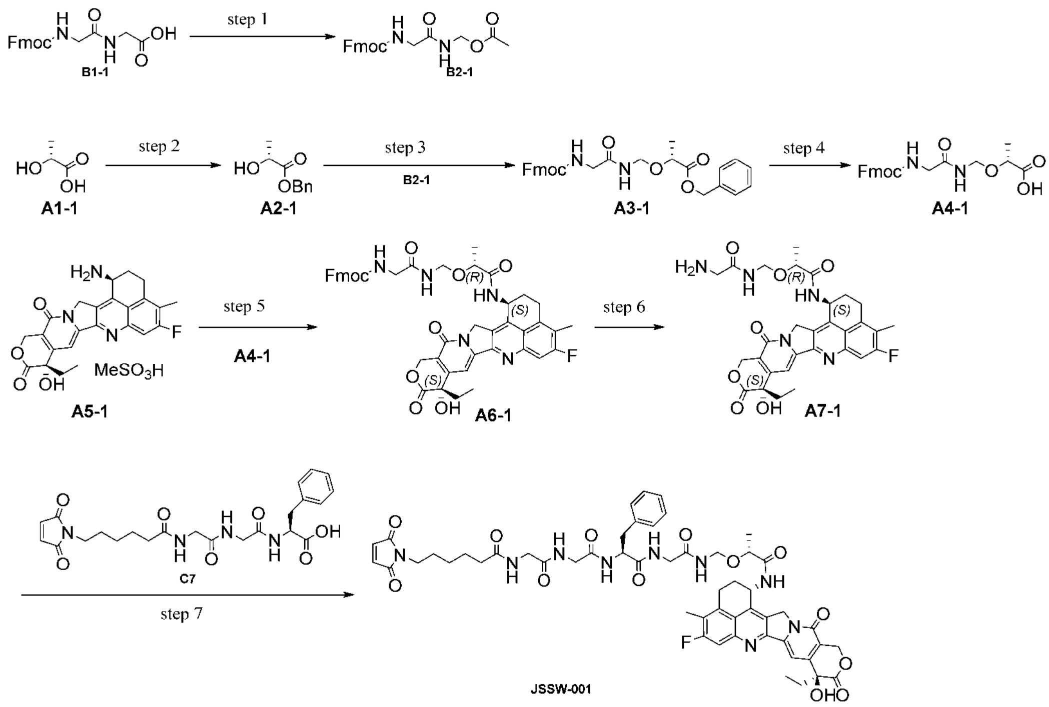


Step 1: Preparation of compound B2-1: B1-1 (1.0g, 2.28mmol), anhydrous tetrahydrofuran (30mL), toluene (10mL), pyridine (0.5mL), and lead tetraacetate (1.83g, 4.12mmol) were added to a 100mL three-neck flask. The solution turned orange and was heated under reflux. After one hour, the solution became colorless, and a white solid was formed. After a three-hour reaction, the solution was filtered using celite, the solid was rinsed with ethyl acetate, and the filtrate was dried using a rotary evaporator. The obtained crude product was purified by column chromatography to produce a solid (640mg). LC-MS$[M+Na]^+$: m/z 391.1.

Step 2: Preparation of compound A2-1: A1-1 (2.7g, 30.0mmol) and N,N-dimethylformamide (30mL) were added to a 100mL three-neck flask, followed by the addition of potassium carbonate (4.17g, 30.0mmol) and benzyl bromide (2.4mL, 20.0mmol). After reacting at room temperature for 14 hours, the reaction mixture was added dropwise into water. Extraction was carried out using ethyl acetate, and the organic layers were combined. The combined organic layers were washed with saturated brine, dried over anhydrous magnesium sulfate, filtered, and concentrated. The crude product was purified by column chromatography (PE/EA=3:1) to produce 2.61g of the target compound as a colorless liquid.

$^1H$ NMR (400 MHz, $CDCl_3$): δ 7.33-7.41 (m, 5H), 5.21 (s, 2H), 4.29-4.35 (m, 1H), 2.77 (d, J = 5.6 Hz, 1H), 1.44 (d, J = 6.8 Hz, 3H).

Step 3: Preparation of compound A3-1: A2-1 (720mg, 4.0mmol), B2-1 (368mg, 1.0mmol), and dichloromethane (5mL) were added to a 100mL three-neck flask. Pyridinium 4-methylbenzenesulfonate (75mg, 0.30mmol) was then added. The reaction mixture was heated to reflux overnight. After the reaction was complete, ethyl acetate (20mL) was added to the reaction mixture, which was then washed with water three times and dried over anhydrous magnesium sulfate. The magnesium sulfate was removed by filtration, and the solution was concentrated. The obtained crude product was purified by column chromatography (PE/EA=2:1) to produce a white solid A3-1 (290mg).

LC-MS$[M+Na]^+$: m/z 511.1. $^1H$ NMR (400 MHz, $CDCl_3$): δ 7.77 (d, J = 7.2 Hz, 2H), 7.58 (d, J = 7.6 Hz, 2H), 7.40 (t, J = 7.6 Hz, 2H), 7.26-7.37 (m, 6H), 6.70 (brs, 1H), 5.12-5.30(m, 3H), 4.76-4.89 (m, 2H), 4.45 (d, J = 6.8 Hz, 1H), 4.19-4.27 (m, 2H), 3.69-3.85 (m, 2H), 1.41 (d, J = 6.8 Hz, 3H).

Step 4: Preparation of compound A4-1: A3-1 (291mg, 0.60mmol) dissolved in tetrahydrofuran:ethyl acetate (4mL/2mL) was added to a 100mL three-neck flask, along with Pd/C (10% w/w, 60mg). After purging with hydrogen gas three times, a hydrogen balloon was attached, and the mixture was stirred overnight. After the reaction was complete, the mixture was filtered through celite, rinsed with methanol, and the organic layer was concentrated to produce the product A4-1. LC-MS$[M+Na]^+$: m/z 399.1.

Step 5: Preparation of compound A6-1: A4-1 (50mg, 0.13mmol, 1.3eq), exatecan mesylate (A5-1) (50mg, 0.049mmol, 1.0eq), 2-(7-azabenzotriazol-1-yl)-N,N,N',N'-tetramethyluronium hexafluorophosphate (48mg, 0.13mmol, 1.3eq), and N,N-diisopropylethylamine (36mg, 0.28mmol, 3.0eq) were dissolved in N,N-dimethylformamide (2mL) in a 25mL single-neck flask. The mixture was stirred overnight at room temperature. It was then diluted with ethyl acetate and washed with semi-saturated citric acid, followed by washing with brine, sodium bicarbonate solution, and brine again. The

organic layer was dried over anhydrous sodium sulfate and filtered. The anhydrous sodium sulfate was removed, and the filtrate was concentrated. Purification was carried out using prep-TLC with a developer system of (DCM/MeOH=15:1) to produce the product A6-1 (56mg). LC-MS$[M+H]^+$: m/z 816.3.

Step 6: Preparation of compound A7-1: A6-1 (56mg, 0.069mmol), N,N-dimethylformamide (2mL), and piperidine (12mg, 0.14mmol) were added to a 25mL single-neck flask and stirred for 2 hours. The solution was then concentrated to produce a white solid. LC-MS$[M+H]^+$: m/z 594.3.

Step 7: Preparation of compound JSSW-001: A7-1 (0.069mmol), C7 (66mg, 0.14mmol), 2-(7-azabenzotriazol-1-yl)-N,N,N',N'-tetramethyluronium hexafluorophosphate (53mg, 0.14mmol), and N,N-diisopropylethylamine (36mg, 0.28mmol) were dissolved in N,N-dimethylformamide (2mL) in a 25mL single-neck flask. The mixture was stirred for 2 hours at room temperature. It was then diluted with ethyl acetate (30mL) and washed with semi-saturated citric acid, followed by washing with brine, sodium bicarbonate solution, and brine again. The organic layer was dried over anhydrous sodium sulfate and filtered. The anhydrous sodium sulfate was removed, and the filtrate was concentrated. Purification was carried out using prep-TLC with a developer system of (DCM/MeOH=15:1) to produce the product JSSW-001 (21mg).

LC-MS$[M+H]^+$: m/z 1048. $^1H$ NMR (400 MHz, DMSO-d6): δ 8.63 (t, J = 6.4 Hz, 1H), 8.53 (d, J = 8.8 Hz, 1H), 8.28 (t, J = 6.0 Hz, 1H), 8.03-8.10 (m, 2H), 7.98 (t, J = 6.0 Hz, 1H), 7.77 (d, J = 11.2 Hz, 1H), 7.30 (s, 1H), 7.14-7.25 (m, 5H), 6.99 (s, 2H), 6.51 (s, 1H), 5.56-5.62 (m, 1H), 5.40 (s, 2H), 5.08-5.23 (m, 2H), 4.66-4.71 (m, 1H), 4.42-4.56 (m, 2H), 4.09-4.15 (m, 1H), 3.54-3.76 (m, 7H), 3.09-3.27 (m, 1H), 2.97-3.02 (m, 1H), 2.67-2.77 (m, 1H), 2.38 (s, 3H), 2.16-2.21 (m, 2H), 2.05-2.10 (m, 2H), 1.83-1.89 (m, 2H), 1.37-1.46 (m, 7H), 1.14-1.33 (m, 4H), 0.87 (t, J = 7.2 Hz, 3H).

Scheme II:

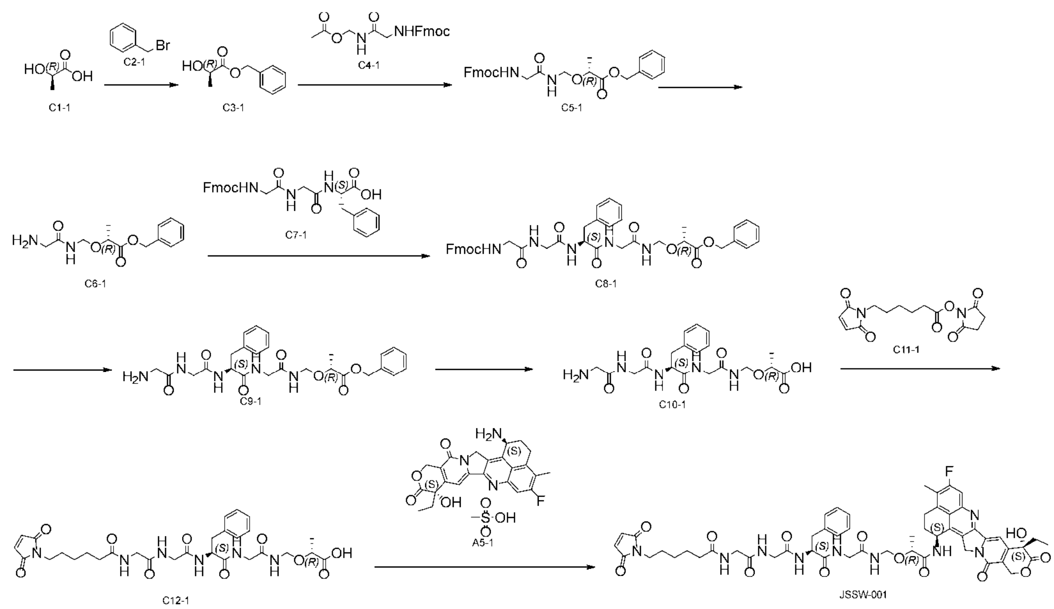


Step 1: Compound C1-1 (8g, 88.86mmol) and C2-1 (22.66g, 133.33mmol) were added to a 250mL reaction flask, followed by the addition of dimethylacetamide (DMAc) (40mL, 5 v/w). The mixture was stirred until dissolved completely, and the temperature was controlled to 0-10°C. Then, N,N-diisopropylethylamine (DIEA) (34.45g, 266.6mmol) was added dropwise. After the dropwise addition, the mixture was allowed to warm naturally to about 25°C and stirred for 16 hours at this temperature. After TLC monitoring showed the complete reaction of the starting materials, the reaction mixture was poured into 120mL of ice water. Methyl tert-butyl ether (MTBE) (40mL) was added, and the mixture was stirred, allowed to stand for phase separation. The aqueous phase was extracted four times with MTBE (40mL*4). The organic phases were combined and washed once with 0.5M hydrochloric acid solution (40mL), once with water (40mL), and twice with saturated brine (40mL*2), dried over anhydrous sodium sulfate, filtered, concentrated, and purified by wet loading and column chromatography using a gradient system of petroleum ether:ethyl acetate = 30:1→20:1→10:1 to produce a light yellow liquid C3-1. LC-MS$[M+Na]^+$: m/z 203.

Step 2: Under nitrogen protection, C4-1 (6g, 16.29mmol, 1.0eq) and C3-1 (3.23g, 17.92mmol, 1.1eq) were dissolved in tetrahydrofuran (THF) (60mL, 10V/w). The temperature was lowered to 10-15°C, and p-toluenesulfonic acid (TsOH) (281mg, 1.63mmol, 0.1eq) was added. After the completion of the addition, the reaction was carried out at 14-18°C for 4 hrs under TLC monitoring. After the reaction was complete, the reaction mixture was added to ice water (60mL) and extracted with ethyl acetate (EA) (60mL*3). The organic phases were combined and washed with saturated aqueous $NaHCO_3$ solution (60mL), water (60mL*2), and saturated brine (60mL), dried over anhydrous sodium sulfate, filtered,

concentrated by evaporating to dryness, and purified by column chromatography using a gradient system of n-hexane:ethyl acetate = 10:1→5:1→3:1→2:1→1:1 to produce a colorless oily substance C5-1. LC-MS[M+Na]$^+$: m/z 511.

Step 3: Under nitrogen protection, C5-1 (2.8g, 5.74mmol, 1.0eq) was dissolved in DMAc (28mL, 10V/w). The temperature was lowered to 14-18°C, and 1,8-diazabicyclo[5.4.0]undec-7-ene (DBU) (436.6mg, 2.87mmol, 0.5eq) was added dropwise. The mixture was stirred at this temperature for 1.5h. TLC monitoring showed the complete reaction of the starting materials. The temperature was then lowered to 0-10°C, and; pyridinium 4-methylbenzenesulfonate (PPTS) (721.23mg, 2.87mmol, 0.5eq), 1-(3-dimethylaminopropyl)-3-ethylcarbodiimide hydrochloride (EDCI) (1.1g, 5.74mmol, 1.0eq), 1-hydroxybenzotriazole (HOBT) (775mg, 5.74mmol, 1.0eq), and C7-1 (2.45g, 4.88mmol, 0.85eq) were added. After the completion of the addition, the reaction was carried out at 0-10°C for 3-4 hrs under TLC monitoring until C7-1 was completely reacted. The reaction mixture was added to ice water (100mL) and extracted with 2-methyl tetrahydrofuran (2-Me THF) (100mL*3). The organic phases were combined and washed with 0.5M hydrochloric acid (150mL*2), saturated aqueous $NaHCO_3$ solution (100mL*3), water (100mL), and saturated brine (100mL), dried over anhydrous sodium sulfate, filtered, concentrated by evaporating to dryness, and purified by column chromatography using a gradient system of DCM→DCM:MeOH = 80:1→60:1→50:1→40:1→30:1→20:1 to produce a white foamy solid C8-1 (3.1g, yield 72%). LC-MS[M+Na]$^+$: m/z 772.

Step 4: Under nitrogen protection, C8-1 (3.1g, 4.14mmol, 1.0eq) was dissolved in dichloromethane (DCM) (46.5mL, 15V/w) at a room temperature of 25-30°C. DBU (314.6mg, 2.07mmol, 0.5eq) was added dropwise, and the mixture was stirred at this temperature for 16h. TLC monitoring showed the complete reaction of the starting materials. The reaction mixture was directly subjected to wet column chromatography using a gradient system of DCM-DCM:MeOH = 50:1→30:1→20:1→10:1 to produce a white foamy solid C9-1. LC-MS[M+H]$^+$: m/z 528.

Step 5: Under nitrogen protection, C9-1 (1.7g, 3.2mmol, 1.0eq) was dissolved in water (25.5mL, 15V/w) and tert-butanol (8.5mL, 5V/w). The mixture was stirred until it dissolved completely. The atmosphere was replaced with nitrogen gas once, and Pd/C (0.34g, 20% w/w) was added. The atmosphere was replaced with hydrogen gas three times. The hydrogenation reaction was carried out at a room temperature of 25-30°C for 12hrs. LCMS monitoring showed the complete reaction of the starting materials. The reaction mixture was filtered, and the filter cake was washed with water (25mL*2). The filtrate was filtered again, and the tert-butanol was removed by concentration. The aqueous phase was directly lyophilized to produce a white powdery solid C10-1 (1.5g in total, yield 100%). LC-MS[M+H]$^+$: m/z 438.

Step 6: Under nitrogen protection, C10-1 (1.4g, 3.2mmol, 1.0eq) was dissolved in acetonitrile (14mL, 10V/w) and water (28mL, 20V/w). C11-1 (1.86g, 6.097mmol, 1.1eq) was added, and the mixture was stirred until completely dissolved. The temperature was lowered to 0-10°C, and DIEA (330.24mg, 2.56mmol) was added dropwise. After the dropwise addition, the mixture was stirred to react at this temperature for 16h. LC-MS monitoring showed the complete reaction of the starting materials. A buffer solution was prepared by dissolving $Na_2HPO_4$ (260mg) and $NaH_2PO_4$ (5.6g) in 20mL of water under stirring, and the prepared buffer solution was cooled to 0-5°C. The reaction mixture was poured into the cooled buffer solution and extracted with DCM/isopropanol = 4/1 (50mL*4). The organic phases were combined, dried, filtered, concentrated by evaporating to dryness, and purified by column chromatography using a gradient system of DCM→DCM:MeOH = 50:1→30:1→20:1→15:1 to produce a yellow solid C12-1.

Step 7: Under nitrogen protection, A5-1 (547.4mg, 1.03mmol, 1.0eq) was dissolved in 5% anhydrous sodium sulfate in water (6.5mL) and THF (7.8mL). The mixture was stirred, but did not dissolve completely. The temperature was lowered to 0-10°C. Then, N-methylmorpholine (NMM) (104mg, 1.03mmol, 1.0eq) was added. After the completion of the addition, the reaction was allowed to proceed at this temperature for 1 hour. C12-1 (650mg, 1.03mmol, 1.1eq), EDCI (296.2mg, 1.545mmol, 1.5eq), and ethyl cyanoglyoxylate-2-oxime (73.9mg, 0.52mmol, 0.5eq) were added sequentially. After the completion of the addition, the reaction was allowed to proceed at 0-10°C for 4-5 hours. TLC monitoring showed the complete reaction of the starting materials. The reaction mixture was then added to 0.1M hydrochloric acid (13mL, 20V/w) in an ice bath, extracted with 2-methyl tetrahydrofuran (2-Me THF) (13mL*3), and the organic phases were combined, washed once with 0.05M hydrochloric acid (13mL, 20V/w) and twice with water (13mL*2), then dried, filtered, concentrated by evaporating to dryness, and purified by column chromatography using a gradient system of DCM→DCM:MeOH=50:1→30:1→20:1→15:1→12:1 to produce a yellow foamy solid (JSSW-001). LC-MS[M+H]$^+$: m/z 1048.

Example 2: Preparation of Antibodies

SWY2110 was obtained by purifying the expression product after transiently transfecting HEK293 cells with plasmids encoding the light and heavy chain genes using known technology.

SWY2110 and EGFR protein were modeled and docked using computer simulation-assisted technology to obtain an antigen-antibody complex. Amino acids within 3Å of the complex underwent D, E, and H scanning, which produced several pH-dependent antibodies that had a reduced affinity at pH 7.4 but basically had an unchanged affinity or had a lower decrease in affinity at pH 6.0. Several obtained antibody sequence genes were cloned into mammalian expression vectors and expressed through transfection of HEK-293 cells. After the completion of the expression, the expression supernatants were collected, and the antibodies were purified using an AKTA system equipped with a Protein A

pre-packed column to produce the desired antibodies SWY2111, SWY2112, and SWY2113.

Example 3: Preparation of an antibody-drug conjugate SWY2110-JSSW-001 ADC:

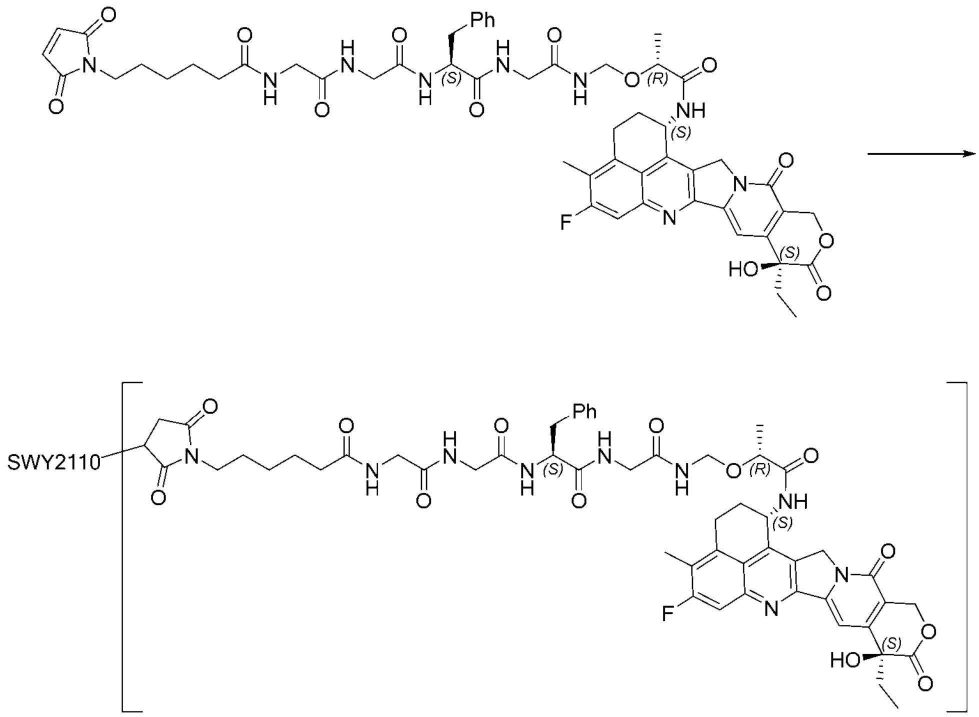

7.12

Step 1. Reduction of antibody:

The SWY2110 antibody medium, which was obtained by purification after the expression of plasmids encoding light and heavy chain genes that were transiently transfected into HEK293 cells using known technology, was replaced with PBS6.0/EDTA to prepare an antibody solution with a concentration of 10 mg/mL.

This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90mg/ml, at pH = 5.5. The non-conjugated linker-drug and other low molecular weight reagents were removed to produce a purified antibody-drug conjugate (SWY2110-JSSW-001 ADC, DAR=7.12). The DAR value result is shown in Figure 1.

Step 4. Determination of Drug-Antibody Ratio (DAR) of Antibody-Drug Conjugate:

Dithiothreitol with a final concentration of 20 mM was added to 2 mg/kg antibody-drug-conjugate, and the mixture was incubated in a 37°C water bath for 30 minutes. The sample, in which the disulfide bonds between antibody-drug conjugate chains were cleaved, was then used for HPLC analysis. The used HPLC system was the Agilent Technologies 1260 Infinity HPLC, and the used chromatograph column was the PLRP-S column (5 μm particle size; 2.1 mm×50 mm; Agilent Technologies). The column temperature was set at 80°C. The mobile phase A was 0.1% trifluoroacetic acid (TFA) in water, and the mobile phase B was 0.1% TFA in acetonitrile. The injection volume was 10μL, and the gradient program was as follows: 0-3 minutes at 27%-27%, 3-8 minutes at 27%-35%, 8-25 minutes at 35%-43%, 25-26 minutes at 43%-95%, 26-31 minutes at 95%-95%, 31-32 minutes at 95%-27%, and 32-40 minutes at 27%-27%. Compared to the unconjugated light chain (L0) and heavy chain (H0), the hydrophobicity increased with the number of conjugated drugs for the drug-conjugated light chain (L1, conjugated with one drug) and heavy chains (H1, conjugated with one drug; H2, conjugated with two drugs; H3, conjugated with three drugs). Therefore, the elution followed the order of L0, L1, H0, H1, H2, and H3. The DAR value was calculated based on the peak area at 280 nm.

In the specification and claims of the present application, unless otherwise specified, the DAR value is calculated based on the peak area at 280 nm by HPLC analysis, as described above.

$$\mathrm{DAR} = (\frac{\mathrm{L1}}{\mathrm{L0+L1}} \times 1 + \frac{\mathrm{H1}}{\mathrm{H0+H1+H2+H3}} \times 1 + \frac{\mathrm{H2}}{\mathrm{H0+H1+H2+H3}} \times 2 + \frac{\mathrm{H3}}{\mathrm{H0+H1+H2+H3}} \times 3) \times 2$$

The same method as that for preparing SWY2110-JSSW-001 ADC was used with replacing the antibody SWY2110 with SWY2111, SWY2112, and SWY2113 to obtain SWY2111-JSSW-001 ADC (Dar: 7.48, the DAR value result is shown in Figure 2), SWY2112-JSSW-001 ADC (Dar: 7.51, the DAR value result is shown in Figure 3), SWY2113-JSSW-001 ADC (Dar: 7.50, the DAR value result is shown in Figure 4), respectively:

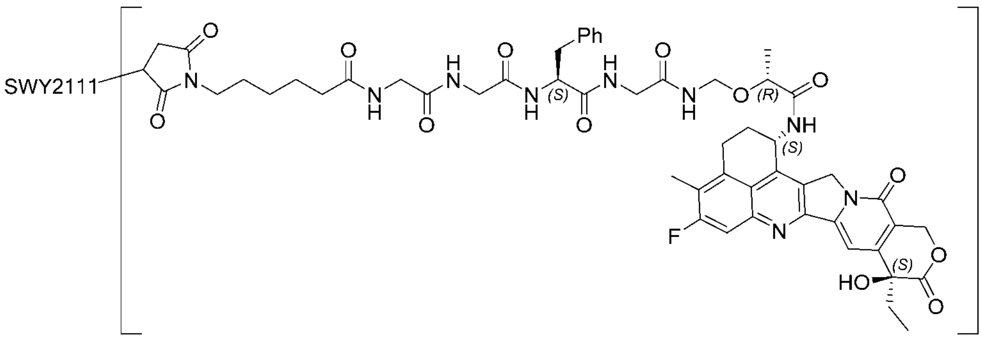

7.48

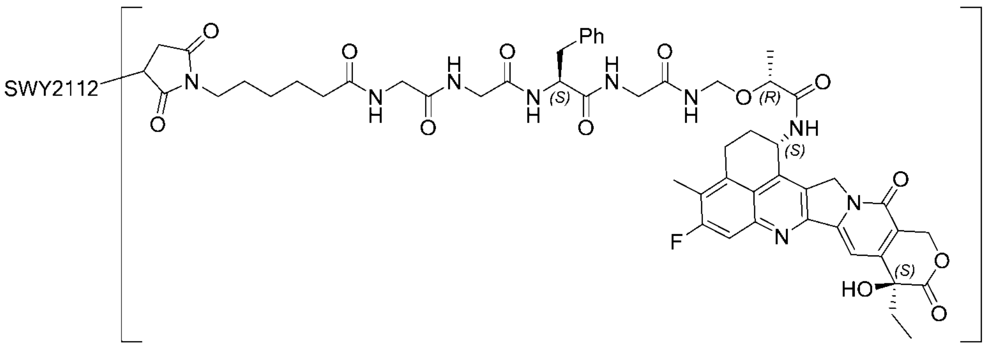

7.51

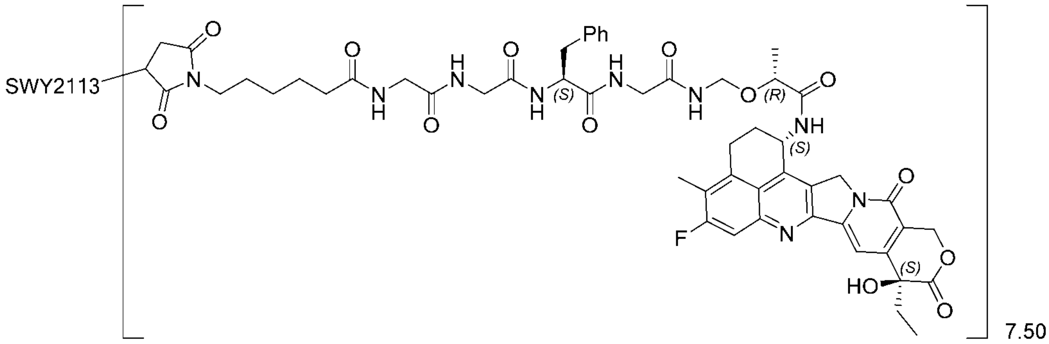

The same method as that for preparing SWY2110-JSSW-001 ADC was used but replacing the antibody JSSW-001 with GGFG-DXD to obtain SWY2110-DXD ADC (DAR: 6.93, the DAR value result is shown in Figure 5):

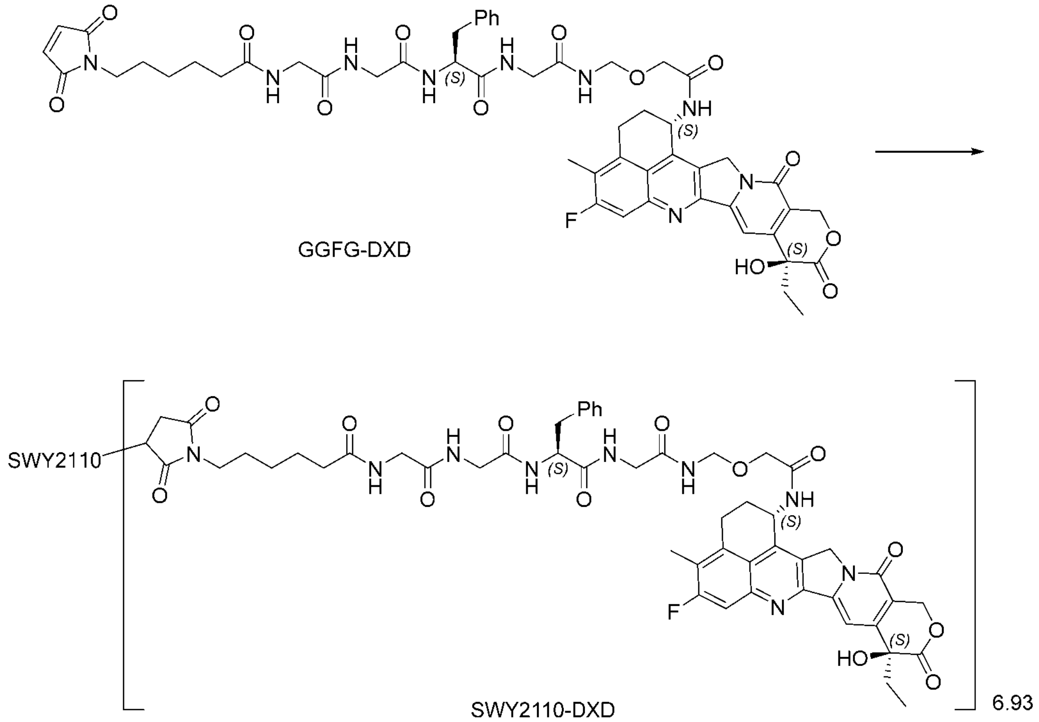

GGFG-DXD

SWY2110-DXD

Preparation of antibody-drug conjugate (SWY2110-VC MMAE ADC, DAR=4.53):

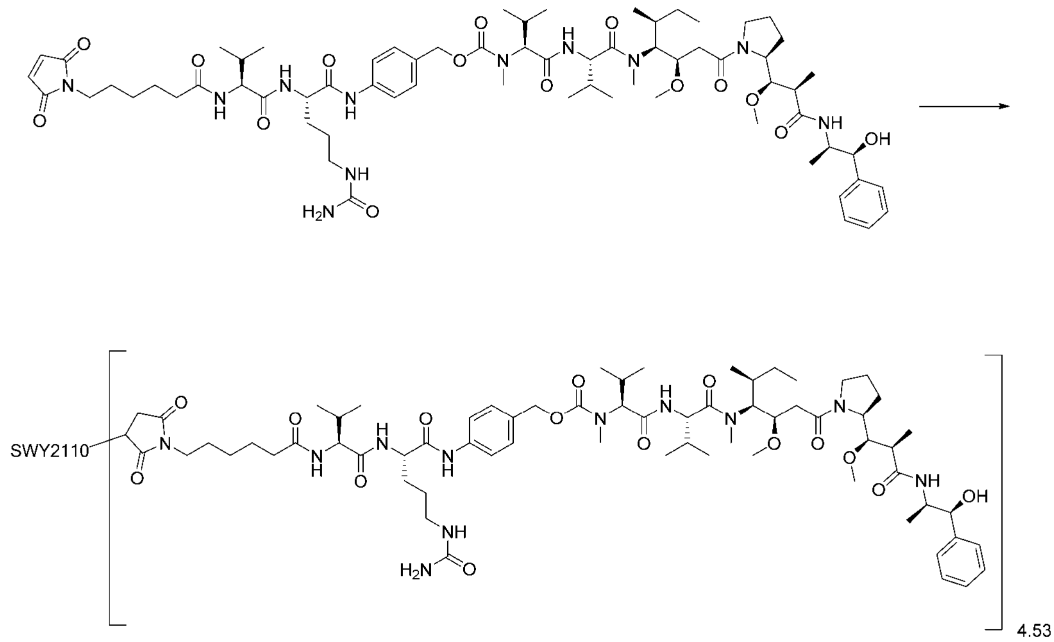

Step 1. Reduction of antibody:

The SWY2110 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd.). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of commercially available mc-vc-PAB-MMAE (CAS No.:646502-53-6, abbrev. Vc MMAE) in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the replacement ratio greater than 1000 times. Thus, the non-conjugated linker-drug compound and other low molecular weight reagents were removed to obtain the purified antibody-drug conjugate (SWY2110-VC MMAE ADC, DAR=4.53).

Step 4. Determination of Drug-Antibody Ratio (DAR) of Antibody-Drug Conjugate:

10 μg SWY2110-Vc MMAE ADC sample was loaded onto the chromatographic column (2.5 μm particle size; 4.6 mm × 10 cm; TSKgel Butyl-NPR). The elution mobile phase A was 20 mM PB, pH 7.0, 1.5 M $(NH_4)_2SO_4$ aqueous solution, and the mobile phase B was 20 mM PB, pH 7.0, 25% isopropanol solution. The flow rate was controlled at 0.8mL/min, the column temperature was 30°C, the detection wavelength was 280 nm, and the gradient program was set as follows: 0-20 minutes, mobile phase B from 0 to 100%; 20-25 minutes, 100% mobile phase B; and 25-30 minutes, 100% mobile phase A. Being non-conjugated was DAR0, being conjugated with 2 small molecules was DAR2, being conjugated with 4 small molecules was DAR4, being conjugated with 6 small molecules was DAR6, and being conjugated with 8 small molecules

was DAR8. Therefore, elution was performed in the order of DAR0, DAR2, DAR4, DAR6, and DAR8. The DAR value was calculated based on the peak area at 280nm, where A represents the percentage of each peak area. A result of the DAR value is shown in Figure 6. $DAR=2\times A_{DAR2}+4\times A_{DAR4}+6\times A_{DAR6}+8\times A_{DAR8}$

Biological Examples

Assay 1: Activity of antibody-drug conjugate in cells in vitro

1. Assay object:

The object of this assay was to detect the inhibitory activity of the antibody-drug conjugate of the present invention on the in vitro proliferation of SK-BR-3 (ATCC/HTB-30) tumor cells, human colorectal cancer cells DiFi, human lung cancer gefitinib-resistant cell PC9-GR, or human lung cancer mutant cell PC-9 (Del19-T790M-C797S). The cells were treated in vitro with compounds of different concentrations. After culture, resazurin was added to read the fluorescence values of ex550nm/em610nm, and the data were fitted with four parameters to obtain the $IC_{50}$ values, thereby calculating the biological activities of the compound.

2. Assay materials and equipment:

Materials

| Product Name | Manufacturer/Item Number |
|---|---|
| SK-BR-3 cell line | ATCC/HTB-30 |
| DiFi cell line | Shanghai Jinmante Biotechnology Co., Ltd |
| PC9-GR cell line | Nanjing Cobioer Biosciences Co., Ltd |
| MTT Assay Kit | Abcam |
| DMEM, high glucose | Hyclone/SH30022.01 |
| 0.25% Trypsin-EDTA | Gibco/25200-072 |
| fetal bovine serum (FBS) | Gibco/16000-044 |
| 100×bispecific antibody | Gibco/15240-062 |
| black walled, transparent-bottom tissue culture plate | Corning/3603 |
| resazurin sodium | Sigma Aldrich/199303-25G |

Equipments

| Name | Model | Manufacturer |
|---|---|---|
| Biosafety cabinet | 1300 series A2 model | Thermo Fisher Scientific |
| $CO_2$ incubator | 3111 model | Thermo Fisher Scientific |
| Inverted microscope | CKX31 | Olympus |
| Microplate reader | Infinite M200 | Tecan Company |
| Micro-oscillator | MM-I model | Shanghai Yarong Biochemistry Instrument Factory |

3. Assay operation procedure 1:

3.1 culture medium: DMEM, 10% FBS, 1×bispecific antibody

3.2 Cell culturing: Frozen SK-BR-3/MDA-MB-468 cells were taken out of liquid nitrogen and revived in a 75cm$^2$ culture vessel for culturing. The cells were grown until the confluency of cells reached >75%, and had undergone at least 3 passages.
3.2.1 If cell passage was not performed, the culture medium should be replaced every 3-4 days.
3.2.2 If the cell expansion was needed, the cells were passaged and inoculated into a larger culture vessel, ensuring that the confluency of cells reached >75% before use in assays.
3.3 Cell Collection: SK-BR-3/MDA-MB-468 cells were collected when the culture vessel was nearly full.
3.3.1 The culture medium was discarded, and the cells were washed with PBS to remove dead cells and residual culture medium.
3.3.2 2-3mL of 0.25% Trypsin-EDTA was added, the culture vessel was gently shaken, and then incubated at 37°C for 2-3 minutes to digest the cells.
3.3.3 5mL of the culture medium was immediately added to the culture vessel and gently pipetted up and down to spread the cells.
3.3.4 The cell suspension was transferred to a sterile centrifuge tube and centrifuged at 200×g for 3 minutes. 3.3.5 The culture medium in the tube was discarded, and 5-10mL of the fresh culture medium was added to resuspend the cells.
3.4 Cell density determination: The cells were counted with a cell counting plate under a microscope. 3.5 Assay plate inoculation
3.5.1 Cells were diluted to $1\times10^5$ cells/mL with the culture medium and seeded at 100μL/well in the assay plate (except rows A and H).
3.5.2 120μL of the culture medium was added to each well in rows A and H as a blank.
3.5.3 The incubation was performed at 37°C with 5% $CO_2$ for 4-6 hrs to allow the cells to adhere to the wall.

3.6 Preparation of diluted compound samples:

The compound was diluted to an initial concentration of 18 μg/ml, and then further diluted by a factor of 3 to obtain a total of 11 gradient solutions. The 12th column was left as a blank control.

3.7 Drug treatment

3.7.1 20μL of drug diluent from each of the 1st to 11th columns of the dilution plate was taken and added to the corresponding column of the assay plate.
3.7.2 20μL of fresh culture medium was added to each well in the 12th column and the A and H rows.
3.7.4 The plate was gently shaken on a plate shaker for 10-15 seconds, and then the culture plate was incubated at 37°C for 3 days.

3.8 Analyses

3.8.1 After incubation, 20μL of 0.03% resazurin (1×PBS diluted) was added to each well and gently shaken for 10-15 seconds.
3.8.2 After incubating at 37°C for 3-4 hours, the plate was read using a microplate reader with the following parameters:

Exciting light: 550nm
Emitting light: 610nm
Integration time: 50
Shaking: 15 seconds, vortex
Reading: top reading
Number of reading/well: 1
Gain: optimization setting (between 35 and 42)

If reading multiple plates, it was ensured that the gain setting was consistent for all plates.
3.8.3 EXCEL was used to plot the data and fit the $IC_{50}$ values for the reference standards and samples.
3.8.3.1 Model 201 was used to plot the data points.
3.8.3.2 For the Fit parameters, the following commands were used:

a. A: Pre-fitted
b. B: Pre-fitted
c. C: Pre-fitted
d. D: Pre-fitted
e. No constraints for all

3.8.3.3 The output parameter C represented the $IC_{50}$ in units of ng/mL.

Assay operation procedure 2:

This assay verified the inhibition effects of SWY2110 monoclonal antibody (SWY2110 mAb), SWY2110-DXD ADC (DAR=6.93), and SWY2110-JSSW-001 ADC (DAR=7.12) on the proliferation of two cell lines, DiFi (human colorectal cancer cells) and PC9-GR (gefitinib-resistant human lung cancer cells). The specific concentration settings were as follows:
DiFi: SWY2110 mAb, SWY2110-DXD ADC (DAR=6.93), and SWY2110-JSSW-001 ADC (DAR=7.12) started at a concentration of 3000 ng/ml, with a 3x serial dilution of the drug, resulting in 10 concentrations: 3000, 1000, 333.33, 111.11, 37.04, 12.35, 4.12, 1.37, 0.46, 0.15 ng/ml, respectively. The drug exposure lasted for 144 hours.
PC9-GR: SWY2110 mAb, SWY2110-DXD ADC (DAR=6.93), SWY2110-JSSW-001 ADC (DAR=7.12) started at a concentration of 1000 ng/ml, with a 5x serial dilution of the drug, resulting in 8 concentrations: 1000, 200, 40, 8, 1.6, 0.32, 0.64, 0.013 ng/ml respectively. The drug exposure lasted for 144 hours. Cells in logarithmic growth phase were seeded in a 96-well plate (100μL/well) at a certain number. After the adherent cells had adhered for 24 hours, 100μL of culture medium containing different concentrations of SWY2110 mAb, SWY2110-DXD ADC (DAR=6.93), or SWY2110-JSSW-001 ADC (DAR=7.12) was added to each well. Three replicate wells were set for each drug concentration, along with corresponding blank wells (only containing culture medium) and normal wells (drug concentration of 0). After 144 hours of drug exposure, a MTT working solution (5 mg/mL) was added to each well (20μL per well). The plates were incubated at 37°C for 4 hours, and then the supernatant was discarded. DMSO (analytical grade) was added (150μL per well), and the plates were shaken on a microplate shaker to mix thoroughly. The plates were then wiped clean, and the optical density (OD) was measured at 550 nm using the microplate reader. The inhibition rate of the cell growth was calculated with the following equation:

$$\text{Inhibition Rate}(\%)=(\text{OD Value}_{\text{normal well}}-\text{OD Value}_{\text{drug well}})/(\text{OD Value}_{\text{normal well}}-\text{OD Value}_{\text{blank well}})\times 100\ \%$$

Based on the inhibition rates at different concentrations, the half-maximal inhibitory concentration ($IC_{50}$) of the drug was calculated using SPSS 19.0. The results are shown in Table 2 and Figures 9 and 10.

Table 2: $IC_{50}$ value of SWY2110-ADC against two cell lines (ng/mL)

| Cell | SWY2110 mAb | SWY2110-DXD ADC (DAR=6.93) | SWY2110-JSSW-001 ADC (DAR=7.12) |
|---|---|---|---|
| DiFi | 43.94 | 14.85 | 15.84 |
| PC9-GR | -- | 1.00 | 1.12 |
| wherein "--" represents not showing an anti-cell proliferation effect. | | | |

It can be seen from the data in Table 2 that the antibody-drug conjugates SWY2110-JSSW-001 ADC (DAR=7.12) and SWY2110-DXD ADC (DAR=6.93) of the present invention had similar $IC_{50}$ values for EGFR overexpressing cell line DiFi and gefitinib-resistant cell line PC9-GR, and showed better anti-cell proliferation effect compared to SWY2110 mAb.

Assay 2: Efficacy Assay of SWY2110-ADC on human colorectal cancer DiFi xenograft tumor in NU/NU mice

1. Assay animals

NU/NU mice, 5-6 weeks old, 14 mice

2. Assay object

To investigate the anti-tumor effect of two SWY2110-ADC drugs in vivo using a human colorectal cancer DiFi cell

xenograft tumor model in NU/NU mice.

3. Drug dosage and grouping

Table 3: Table of animal grouping and dosage

| Group | Animal Number | Dose (mg/kg) | Administration Route/Frequency |
|---|---|---|---|
| Vehicle | 5 | 0 | i.v./once |
| SWY2110-Vc MMAE ADC (DAR=4.53) | 4 | 0.5 | i.v./once |
| SWY2110-JSSW-001 ADC (DAR=7.12) | 5 | 0.5 | i.v./once |
| Note: i.v.: intravenous injection. | | | |

4. Assay method

In this assay, human colorectal cancer DiFi cells were used to construct a xenograft tumor model in nude mice. When the tumor volume reached approximately 110 $mm^3$, the animals were evenly divided into 3 groups (d0) based on tumor volume according to Table 3, with 5 animals in the vehicle group and the SWY2110-JSSW-001 ADC (DAR=7.12) group, and 4 animals in the SWY2110-Vc MMAE ADC (DAR=4.53) group. Intravenous administration was given once with an administration volume of 10mL/kg. The vehicle control group (Vehicle) received a 0.9% sodium chloride injection. Observation was made for 27 days after the administration. The inhibition effects of two SWY2110-ADC drugs in the table on the human colorectal cancer DiFi xenograft tumor in nude mice were compared.

5. Observation indicator

5.1 Evaluation indicator

(1) Tumor volume:

$$V = 1/2 \times A \times B^2$$

(2) Relative tumor volume:

$$RTV = \frac{TV_{nd}}{TV_{0d}}$$

(3) Relative tumor volume proliferation rate:

$$\frac{T}{C}\% = \frac{\text{Drug Group } RTV_{xnd}}{\text{Vehicle Group } RTV_{xnd}} \times 100\%$$

(4) Tumor inhibition rate:

$$TGI\% = [1 - \frac{TV_{Xn} - TV_{X0}}{TV_{Mn} - TV_{M0}}] \times 100\%$$

Note: V: Tumor volume
A: tumor length
B: tumor width
RTV: relative tumor volume
$TV_{nd}$: tumor volume on day n
$TV_{0d}$: tumor volume on day 0

$RTV_{xnd}$: average relative tumor volume on day n
$TV_{Xn}$: average tumor volume of drug group on day n
$TV_{X0}$: average tumor volume of drug group on day 0
$TV_{Mn}$: average tumor volume of vehicle group on day n
$TV_{M0}$: average tumor volume of vehicle group on day 0

6 Result

6.1 Tumor volume

At the end of the assay, compared with the vehicle group, both SWY2110-Vc MMAE ADC (DAR=4.53) (P<0.05) and SWY2110-JSSW-001 ADC (DAR=7.12) (P<0.05) could significantly inhibit the volume of the xenograft tumor. The tumor inhibition rate on day 27 of the SWY2110-JSSW-001 ADC (DAR=7.12) group reached as high as 74.6%, followed by the SWY2110-Vc MMAE ADC (DAR=4.53) group with a tumor inhibition rate of 69.7%. Since the toxicity of the JSSW-001 small molecule was lower than that of Vc MMAE, it was suggested that SWY2110-JSSW-001 ADC (DAR=7.12) can achieve a more efficient and less toxic therapeutic effect. For details, see Figure 7 and Table 4.

Table 4: Tumor parameters of each group 27 days after administration of SWY2110-ADC

| Group | Animal Number | Dose (mg/kg) | Tumor Volume ($mm^3$) | TGI (%) Day27 |
|---|---|---|---|---|
| Vehicle | 5 | 0 | 763.1±293.8 | --- |
| SWY2110-Vc MMAE ADC (DAR=4.53) | 4 | 0.5 | 306.5±332.4* | 69.7 |
| SWY2110-JSSW-001 ADC (DAR=7.12) | 5 | 0.5 | 275.5±297.5* | 74.6 |
| *: compared with the vehicle group, P<0.05 | | | | |

Assay 3 Efficacy assay of SWY2110-ADC on human lung cancer PC9-GR (gefitinib-resistant cell) xenograft tumor in NU/NU mice

1. Assay animals

NU/NU mice, 5-6 weeks old, 30 mice

2. Assay object

To investigate the anti-tumor effect of SWY2110 monoclonal antibody and three SWY2110-ADC drugs in vivo using a human lung cancer PC-9-GR cell (gefitinib-resistant cell) xenograft tumor model in NU/NU mice.

3. Drug dosage and grouping

Table 5: Table of animal grouping and dosage

| Group | Animal Number | Dose (mg/kg) | Administration Route/Frequency |
|---|---|---|---|
| Vehicle | 6 | 0 | i.v./qw*4w |
| SWY2110 mAb | 6 | 1 | i.v./qw*4w |
| SWY2110-Vc MMAE ADC (DAR=4.53) | 6 | 1 | i.v./qw*4w |
| SWY2110-DXD ADC (DAR=6.93) | 6 | 1 | i.v./qw*4w |
| SWY2110-JSSW-001 ADC (DAR=7.12) | 6 | 1 | i.v./qw*4w |
| Note: i.v.: intravenous injection, qw*4w: administration once weekly for 4 weeks. | | | |

4. Assay method

In this assay, human lung cancer PC9-GR cells (gefitinib-resistant cells) were used to construct a xenograft tumor

model in nude mice. When the tumor volume reached approximately 110 $mm^3$, the animals were evenly divided into 5 groups (d0) based on tumor volume according to Table 5, with 6 animals in each group. Intravenous administration was given once weekly for 4 weeks, with an administration volume of 10mL/kg. The vehicle control group (Vehicle) received a 0.9% sodium chloride injection. The assay was terminated on day 28 after the administration. The inhibition effects of SWY2110 monoclonal antibody and three SWY2110-ADC drugs on human lung cancer PC9-GR (Gefitinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, compared with the vehicle group, SWY2110-Vc MMAE ADC (DAR=4.53) (P<0.05), SWY2110-DXD ADC (DAR=6.93) (P<0.05), and SWY2110-JSSW-001 ADC (DAR=7.12) (P<0.001) all exhibited inhibition effects on the volume of the xenograft tumor. The antibody SWY2110 showed poor tumor inhibition effect, with a tumor inhibition rate of only 20.3%. The SWY2110-JSSW-001 ADC (DAR=7.12) group had the most significant inhibition effect on the xenograft tumor volume of PC9-GR, with a tumor inhibition rate as high as 77.7%. The tumor inhibition rates of SWY2110-Vc MMAE ADC (DAR=4.53) and SWY2110-DXD ADC (DAR=6.93) were 40.4% and 50.2%, respectively. SWY2110-JSSW-001 ADC (DAR=7.12) showed 154% of the tumor inhibition effect of SWY2110-DXD ADC (DAR=6.93) on the PCR-GR, and SWY2110-JSSW-001 ADC (DAR=7.12) showed 192% of the tumor inhibition effect of SWY2110-Vc MMAE ADC (DAR=4.53) on PCR-GR, suggesting that SWY2110-JSSW-001 ADC (DAR=7.12) still had a significant inhibition effect on gefitinib-resistant tumor cells. For details, see Figure 8 and Table 6.

Table 6: Tumor parameters of each group 28 days after administration of SWY2110-ADC

| Group | Animal Number | Dose (mg/kg) | Administration Route/Frequency | Tumor Volume ($mm^3$) | TGI (%) Day28 |
|---|---|---|---|---|---|
| Vehicle | 6 | 0 | i.v./qw*4w | 761.4±272.6 | --- |
| SWY2110 mAb | 6 | 1 | i.v./qw*4w | 628.9±290.0 | 20.3 |
| SWY2110-Vc MMAE ADC (DAR=4.53) | 6 | 1 | i.v./qw*4w | 498.0±179.1* | 40.4 |
| SWY2110-DXD ADC (DAR=6.93) | 6 | 1 | i.v./qw*4w | 434.6±136.3* | 50.2 |
| SWY2110-JSSW-001 ADC (DAR=7.12) | 6 | 1 | i.v./qw*4w | 255.1±117.7*** | 77.7 |
| *: compared with the vehicle group, P<0.05, ***: compared with the vehicle group, P<0.001 | | | | | |

Assay 4: Efficacy assay of SWY2110-ADC on human lung cancer PC9-AR (PC9-Del19/T790M/C797S, osimertinib-resistant cell) xenograft tumor

1. Assay animals

Balb/c nude mice, 5-6 weeks old, 24 mice

2. Assay object

To investigate the anti-tumor effect of three SWY2110-ADC drugs in vivo using a human lung cancer PC9-Del19/T790M/C797S cells (osimertinib-resistant cell, hereinafter referred to as PC9-AR) xenograft tumor model in NU/NU mice.

3. Drug dosage and grouping

Table 7: Table of animal grouping and dosage

| Group | Animal Number | Dose (mg/kg) | Administration Route/Frequency |
|---|---|---|---|
| Vehicle | 6 | 0 | i.v./once |
| SWY2110-Vc MMAE ADC (DAR=4.53) | 6 | 3 | i.v./once |
| SWY2110-DXD ADC (DAR=6.93) | 6 | 3 | i.v./once |
| SWY2110-JSSW-001 ADC (DAR=7.12) | 6 | 3 | i.v./once |
| Note: i.v.: intravenous injection, i.v./once: administration once. | | | |

4. Assay method

In this assay, human lung cancer PC9-Del19/T790M/C797S cells (osimertinib-resistant cells) were used to construct a xenograft tumor model in nude mice. When the tumor volume reached approximately 110 $mm^3$, the animals were evenly divided into 4 groups (d0) based on tumor volume according to Table 7, with 6 animals in each group. Intravenous administration was given once, with an administration volume of 10mL/kg. The vehicle control group (Vehicle) received a 0.9% sodium chloride injection. The assay was terminated on day 24 after the administration. The inhibition effects of three SWY2110-ADC drugs on human lung cancer PC9-Del19/T790M/C797S (osimertinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, compared with the vehicle group, SWY2110-Vc MMAE ADC (DAR=4.53) (P<0.001), SWY2110-DXD ADC (DAR=6.93) (P<0.001), and SWY2110-JSSW-001 ADC (DAR=7.12) (P<0.001) all exhibited inhibition effects on the volume of the xenograft tumor. The SWY2110-JSSW-001 ADC (DAR=7.12) group demonstrated the most significant inhibitory effect on the PC9-AR xenograft tumor volume, achieving a tumor inhibition rate of as high as 97.5%. The tumor inhibition rates of SWY2110-Vc MMAE ADC (DAR=4.53) and SWY2110-DXD ADC (DAR=6.93) were 89.7% and 76.2% respectively. SWY2110-JSSW-001 ADC (DAR=7.12) showed 128% of the tumor inhibition effect of SWY2110-DXD ADC (DAR=6.93), and SWY2110-JSSW-001 ADC (DAR=7.12) showed 109% of the tumor inhibition effect of SWY2110-Vc MMAE ADC (DAR=4.53), suggesting that SWY2110-JSSW-001 ADC (DAR=7.12) still had a significant inhibition effect on osimertinib-resistant tumor cells. For details, see Figure 11 and Table 8.

Table 8: Tumor parameters of each group 24 days after administration of SWY2110-ADC

| Group | Animal Number | Dose (mg/kg) | Administration Route/Frequency | Tumor Volume ($mm^3$) | TGI (%) Day 24 |
|---|---|---|---|---|---|
| Vehicle | 6 | 0 | i.v./once | 1138.3 | --- |
| SWY2110-Vc MMAE ADC (DAR=4.53) | 6 | 3 | i.v./once | 212.3*** | 89.7 |
| SWY2110-DXD ADC (DAR=6.93) | 6 | 3 | i.v./once | 352.0*** | 76.2 |
| SWY2110-JSSW-001 ADC (DAR=7.12) | 6 | 3 | i.v./once | 131.3*** | 97.5 |
| ***: statistical significance relative to the vehicle group p<0.001 | | | | | |

Assay 5 Efficacy Assay of SWY2110-ADC, SWY2111-ADC, SWY2112-ADC, SWY2113-ADC on human lung cancer NCI-H1975 (human lung cancer cell) xenograft tumor

1. Assay animals

NU/NU mice, 5-6 weeks old, 30 mice

2. Assay object

To investigate the anti-tumor effect of SWY2110-JSSW-001, SWY2111-JSSW-001, SWY2112-JSSW-001, and SWY2113-JSSW-001 drugs in vivo using a human lung cancer NCI-H1975 cell (human lung cancer cell) xenograft tumor model in NU/NU mice.

3. Drug dosage and grouping

Table 9: Table of animal grouping and dosage

| Group | Animal Number | Dose (mg/kg) | Administration Route/Frequency |
|---|---|---|---|
| Vehicle | 6 | - | i.v./once |
| SWY2110-JSSW-001 ADC (DAR=7.12) | 6 | 1 | i.v./once |
| SWY2111-JSSW-001 ADC (DAR=7.48) | 6 | 1 | i.v./once |
| SWY2112-JSSW-001 ADC (DAR=7.51) | 6 | 1 | i.v./once |
| SWY2113-JSSW-001 ADC (DAR=7.50) | 6 | 1 | i.v./once |
| Note: i.v.: intravenous injection, i.v./once: administration once; | | | |

4. Assay method

In this assay, human lung cancer NCI-H1975 cells (human lung cancer cells) were used to construct a xenograft tumor model in nude mice. When the tumor volume reached approximately 110 $mm^3$, the animals were evenly divided into 5 groups (d0) based on tumor volume according to Table 9, with 6 animals in each group. Each dosing group received a single administration at 1mg/kg. Intravenous administration was given once, with an administration volume of 10mL/kg. The vehicle control group (Vehicle) received a 0.9% sodium chloride injection. The assay was terminated on day 21 after the administration. The inhibition effects of SWY2110-JSSW-001, SWY2111-JSSW-001, SWY2112-JSSW-001, and SWY2113-JSSW-001 drugs on human lung cancer NCI-H1975 xenograft tumor in nude mice were compared.

5. Observation indicator

5.1 Evaluation indicator

See Assay 2: the part of 5.1 Evaluation indicator

6 Result

6.1 Tumor volume

At the end of the assay, compared to the vehicle group, SWY2110-JSSW-001 ADC (DAR=7.12), SWY2112-JSSW-001 ADC (DAR=7.51), SWY2111-JSSW-001 ADC (DAR=7.48), SWY2113-JSSW-001 ADC (DAR=7.50) (P<0.001) all demonstrated significant tumor inhibition effects (P<0.001), achieving tumor inhibition rates of 84.1%, 83.0%, 80.9% and 74.2% (P<0.001) respectively. These results indicated that all four ADCs had significant inhibitory effects on human lung cancer NCI-H1975 tumor cells. For details, see Figure 12 and Table 10.

Table 10: Tumor parameters of each group 21 days after administration of SWY2110 to SWY2113-ADCs

| Group | Animal Number | Dose (mg/kg) | Administration Route/Frequency | Tumor volume ($mm^3$) | TGI (%) Day 21 |
|---|---|---|---|---|---|
| Vehicle | 6 | 0 | i.v./once | 852.4±97.8 | --- |
| SWY2110-JSSW-001 ADC (DAR=7.12) | 6 | 1 | i.v./once | 223.3±71.7*** | 84.2 |
| SWY2111-JSSW-001 ADC (DAR=7.48) | 6 | 1 | i.v./once | 248.4±80.3*** | 80.9 |
| SWY2112-JSSW-001 ADC (DAR=7.51) | 6 | 1 | i.v./once | 231.8±106.5*** | 83.0 |
| SWY2113-JSSW-001 ADC (DAR=7.50) | 6 | 1 | i.v./once | 298.4±108.4*** | 74.2 |
| ***: compared with the vehicle group, P<0.001 | | | | | |

Assay 6: Efficacy Assay of SWY2110-ADC, SWY2111-ADC, SWY2112-ADC, SWY2113-ADC on human breast cancer MDA-MB-468 (human breast cancer cell) xenograft tumor

1. Assay animals

NOD-SCID mice, 5-6 weeks old, 28 mice

2. Assay object

To investigate the anti-tumor effect of SWY2110-JSSW-001, SWY2111-JSSW-001, SWY2112-JSSW-001, and SWY2113-JSSW-001 drugs in vivo using a human breast cancer MDA-MB-468 cell (human breast cancer cell) xenograft tumor model in NOD-SCID mice.

3. Drug dosage and grouping

Table 11: Table of animal grouping and dosage

| Group | Animal Number | Dose (mg/kg) | Administration Route/Frequency |
|---|---|---|---|
| Vehicle | 8 | 0 | i.v./once |
| SWY2110-JSSW-001 ADC (DAR=7.12) | 5 | 2 | i.v./once |
| SWY2111-JSSW-001 ADC (DAR=7.48) | 5 | 2 | i.v./once |
| SWY2112-JSSW-001 ADC (DAR=7.51) | 5 | 2 | i.v./once |
| SWY2113-JSSW-001 ADC (DAR=7.50) | 5 | 2 | i.v./once |
| Note: i.v.: intravenous injection, i.v./once: administration once; | | | |

4. Assay method

In this assay, human breast cancer MDA-MB-468 cells (human breast cancer cells) were used to construct a xenograft tumor model in mice. When the tumor volume reached approximately 150 $mm^3$, the animals were divided into 5 groups (d0) based on tumor volume according to Table 11, with 8 animals in the control group and 5 animals in the experimental group. Intravenous administration was given once, with an administration volume of 10mL/kg. The vehicle control group (Vehicle) received a 0.9% sodium chloride injection. The assay was terminated on day 28 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51), and SWY2113-JSSW-001 ADC (DAR=7.50) experimental groups reached 65.8%, 65.1%, 58.3%, and 47.1% (P<0.001) respectively, all demonstrating good tumor inhibition activity. It suggested that four ADC drugs had a significant inhibitory effect on human breast cancer MDA-MB-468 tumor cells. For details, see Figure 13 and Table 12.

Table 12: Tumor parameters of each group 28 days after administration of SWY2110 to SWY2113-ADCs

| Group | Animal Number | Dose (mg/kg) | Administration Route/Frequency | Tumor Volume ($mm^3$) | TGI (%) Day 28 |
|---|---|---|---|---|---|
| Vehicle | 8 | - | i.v./once | 525.0±28.4 | --- |
| SWY2110-JSSW-001 ADC (DAR=7.12) | 5 | 2 | i.v./once | 275.9±50.6*** | 65.8 |
| SWY2111-JSSW-001 ADC (DAR=7.48) | 5 | 2 | i.v./once | 278.8±22.9*** | 65.1 |
| SWY2112-JSSW-001 ADC (DAR=7.51) | 5 | 2 | i.v./once | 304.3±40.6*** | 58.3 |
| SWY2113-JSSW-001 ADC (DAR=7.50) | 5 | 2 | i.v./once | 346.9±46.2*** | 47.1 |
| ***: compared with the vehicle group, P<0.001 | | | | | |

Assay 7 Efficacy Assay of SWY2110-ADC, SWY2111-ADC, SWY2112-ADC, SWY2113-ADC on human colorectal cancer DiFi (human colorectal cancer cell) xenograft tumor

1. Assay animals

NU/NU mice, 5-6 weeks old, 35 mice

2. Assay object

To investigate the anti-tumor effect of SWY2110-JSSW-001, SWY2111-JSSW-001, SWY2112-JSSW-001, and SWY2113-JSSW-001 drugs in vivo using a human colorectal cancer DiFi (human colorectal cancer cell) xenograft tumor model in NU/NU mice.

3. Drug dosage and grouping

Table 13: Table of animal grouping and dosage

| Group | Animal Number | Dose (mg/kg) | Administration Route/Frequency |
|---|---|---|---|
| Vehicle | 7 | - | - |
| SWY2110-JSSW-001 ADC (DAR=7.12) | 7 | 1 | i.v./qw*twice |
| SWY2111-JSSW-001 ADC (DAR=7.48) | 7 | 1 | i.v./qw*twice |
| SWY2112-JSSW-001 ADC (DAR=7.51) | 7 | 1 | i.v./qw*twice |
| SWY2113-JSSW-001 ADC (DAR=7.50) | 7 | 1 | i.v./qw*twice |
| Note: i.v.: intravenous injection, i.v./qw*twice: administration twice weekly; | | | |

4. Assay method

In this assay, human colorectal cancer DiFi cells (human colorectal cancer cells) were used to construct a xenograft tumor model in mice. When the tumor volume reached approximately 100 $mm^3$, the animals were divided into 5 groups (d0) based on tumor volume according to Table 13, with 7 animals in each group. Intravenous administration was given twice, with an administration volume of 10mL/kg. The vehicle control group (Vehicle) received a 0.9% sodium chloride injection. The assay was terminated on day 21 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 86.5%, 82.3%, 83.5%, 76.8% (P<0.001), respectively, all demonstrating good tumor inhibition activity. It suggested that four ADC drugs had a significant inhibitory effect on human colorectal cancer DiFi tumor cells. For details, see Figure 14 and Table 14.

Table 14: Tumor parameters of each group 21 days after administration of SWY2110 to SWY2113-ADCs

| Group | Animal Number | Dose (mg/kg) | Administration Route/Frequency | Tumor Volume ($mm^3$) | TGI (%) Day 28 |
|---|---|---|---|---|---|
| Vehicle | 7 | 0 | - | 562.2±272.8 | --- |
| SWY2110-JSSW-001 ADC (DAR=7.12) | 7 | 1 | i.v./qw*twice | 161.9±41.2*** | 86.5 |
| SWY2111-JSSW-001 ADC (DAR=7.48) | 7 | 1 | i.v./qw*twice | 180.3±98.8*** | 82.3 |
| SWY2112-JSSW-001 ADC (DAR=7.51) | 7 | 1 | i.v./qw*twice | 175.3±36.7*** | 83.5 |
| SWY2113-JSSW-001 ADC (DAR=7.50) | 7 | 1 | i.v./qw*twice | 205.3±155.6*** | 76.8 |
| ***: compared with the vehicle group, P<0.001 | | | | | |

Assay 8: Efficacy Assay of SWY2110-ADC, SWY2111-ADC, SWY2112-ADC, SWY2113-ADC on human lung cancer PC9-GR (human lung cancer gefitinib-resistance acquired cell line) xenograft tumor

1. Assay animals

NU/NU mice, 5-6 weeks old, 30 mice

2. Assay object

To investigate the anti-tumor effect of SWY2110-JSSW-001, SWY2111-JSSW-001, SWY2112-JSSW-001, and SWY2113-JSSW-001 drugs in vivo using a human lung cancer cell PC9-GR xenograft tumor model in NU/NU mice.

3. Drug dosage and grouping

Table 15: Table of animal grouping and dosage

| Group | Animal Number | Dose (mg/kg) | Administration Route/Frequency |
|---|---|---|---|
| Vehicle | 6 | - | - |
| SWY2110-JSSW-001 | 6 | 3 | i.v./once |
| ADC (DAR=7.12) | | | |
| SWY2111-JSSW-001 ADC (DAR=7.48) | 6 | 3 | i.v./once |
| SWY2112-JSSW-001 ADC (DAR=7.51) | 6 | 3 | i.v./once |
| SWY2113-JSSW-001 ADC (DAR=7.50) | 6 | 3 | i.v./once |
| Note: i.v.: intravenous injection, i.v./once: administration once; | | | |

4. Assay method

In this assay, human lung cancer cell PC9-GR cells were used to construct a xenograft tumor model in mice. When the tumor volume reached approximately 110 $mm^3$, the animals were divided into 5 groups (d0) based on tumor volume according to Table 15, with 6 animals in each group. Intravenous administration was given once, with an administration volume of 10mL/kg. The vehicle control group (Vehicle) received a 0.9% sodium chloride injection. The assay was terminated on day 28 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 99.0%, 100.1%, 92.4%, 89.3% (P<0.001), respectively, all demonstrating good tumor inhibition effect. It suggested that four ADC drugs had a significant inhibitory effect on human lung cancer cell PC9-GR tumor cells. For details, see Figure 15 and Table 16.

Table 16: Tumor parameters of each group 28 days after administration of SWY2110 to SWY2113-ADCs

| Group | Animal Number | Dose (mg/kg) | Administration Route/Frequency | Tumor Volume ($mm^3$) | TGI (%) Day 28 |
|---|---|---|---|---|---|
| Vehicle | 6 | - | - | 1159.3 | --- |
| SWY2110-JSSW-001 ADC (DAR=7.12) | 6 | 3 | i.v./once | 124.3*** | 99.0 |
| SWY2111-JSSW-001 ADC (DAR=7.48) | 6 | 3 | i.v./once | 112.9*** | 100.1 |
| SWY2112-JSSW-001 ADC (DAR=7.51) | 6 | 3 | i.v./once | 193.0*** | 92.4 |
| SWY2113-JSSW-001 ADC (DAR=7.50) | 6 | 3 | i.v./once | 225.4*** | 89.3 |
| ***: compared with the vehicle group, P<0.001 | | | | | |

Assay 9: Safety Evaluation Study

In this assay, six appropriately aged crab-eating macaques were selected, with three males and three females, and were intravenously injected with SWY2110-JSSW-001, SWY2111-JSSW-001, and SWY2113-JSSW-001. The

administration regimen was designed as shown in the table below, with intravenous injections once every three weeks for three successive injections. After the administration, a continuous observation was made for 7 days, a general observation was made twice daily, and a detailed observation was made once daily. Changes in body weight were observed before and after administration, and haematological tests were performed. Assay results showed that compared to the ADCs prepared with pH-dependent antibodies (SWY2111-JSSW-001 and SWY2113-JSSW-001), SWY2110-JSSW-001 exhibited reduced toxicity in skin and gastrointestinal tracts, and no obvious target-related toxicity (such as skin ulceration, scabbing, loose stools, etc.), indicating that the pH-modified ADCs had reduced toxicity to normal tissues; however, there was no significant difference in the haematological toxicity caused by the three ADC molecules.

Table 17: Pre-toxicology experiment design scheme

| | | | | |
|---|---|---|---|---|
| Administration frequency | Q3W$\times$3 | | | |
| | Group | D1 | D22 | D43 |
| Administration dosage | Group 1: SWY2110-JSSW-001 | 6mg/kg | 30mg/kg | 30mg/kg |
| | Group 2: SWY2111-JSSW-001 | 6mg/kg | 30mg/kg | 30mg/kg |
| | Group 3: SWY2113-JSSW-001 | 6mg/kg | 30mg/kg | 30mg/kg |
| Animal number | 1 animal/sex/group | | | |

Table 18: Pre-toxicity assay results

| | |
|---|---|
| General observation | Group 1 showed loose stools, skin flakes on limbs, ulceration, and scabbing;<br>Group 2 showed soft stools, mild skin flakes, and no ulceration;<br>Group 3 showed no abnormalities. |
| Body Weight | Group 1 showed a decrease in body weight within 7 days after each administration, with partial recovery before the next administration.<br>Group 2 showed a slight decrease in body weight within 7 days after each |
| | administration, with full recovery before the next administration.<br>Group 3 showed no significant decrease in body weight within 7 days after each administration. |
| Hematology | Group 1 HGB, WBC, LYMP↓;<br>Group 2 HGB, WBC, LYMP↓;<br>Group 3 HGB, WBC, LYMP↓; |
| HGB: haemoglobin; WBC: white blood count; LYMP: lymphocyte absolute number. | |

Clinical Examples

Example 1

1. Study Design

This study was an open-label, multicenter Phase I clinical trial conducted in patients with advanced solid tumors, divided into two stages: the first stage (Stage I) included a dose escalation study and a PK expansion study, and the second stage (Stage II) was a cohort expansion study. The study was aimed at evaluating the safety, tolerability, pharmacokinetic characteristics, and preliminary efficacy of the antibody-drug conjugates of the present invention in patients with advanced solid tumors.

1.1 First stage (Stage I)

1.1.1 Dose escalation study

A dose-escalation study was conducted in patients with advanced solid tumors using accelerated titration and traditional 3+3 designs. The starting dose of the antibody drug conjugate of the present invention was 0.6 mg/kg, and the maximum escalated dose was set at 8.0 mg/kg. The dose-limiting toxicity (DLT) and the maximum tolerated dose (MTD) were determined through the dose escalation study. The dose group settings for the specific dose escalation phase were

determined using a modified Fibonacci method, as detailed in the table below:

Table 19: Dose escalation group settings

| Dose groups | 1 | 2 | 3 | 4 | 5 | 6 |
|---|---|---|---|---|---|---|
| Escalation proportion (%) | - | 200 | 100 | 33.3 | 33.3 | 25 |
| Dose (mg/kg) Q3W | 0.6 | 1.8 | 3.6 | 4.8 | 6.4 | 8.0 |

1.1.2 PK expansion study

Based on the data such as safety, tolerability, and PK from the various dose groups in the dose escalation study, 2 to 3 dose groups were selected for the PK expansion study. Each dose group was expanded to include 8 to 12 subjects (including those subjects from the same dose group in the dose escalation study).

1.2 Second stage (Stage II)

A cohort expansion study was performed using the recommended dose preliminarily determined in the first stage. Subjects were divided into 6 cohorts based on tumor type, as follows:

Cohort A: Colorectal cancer with moderate/high EGFR expression;
Cohort B: Triple-negative breast cancer with moderate/high EGFR expression;
Cohort C: Head and neck squamous carcinoma (including nasopharyngeal carcinoma) with moderate/high EGFR expression;
Cohort D: EGFR-mutant non-squamous non-small cell lung cancer (Nsq-NSCLC);
Cohort E: lung squamous carcinoma (Sq-NSCLC) with moderate/high EGFR expression;
Cohort F: Other advanced solid tumors with moderate/high EGFR expression, such as gastric cancer, esophageal cancer, pancreatic cancer, cervical cancer, biliary tract cancer, non-squamous non-small cell lung cancer, and RAS/BRAF mutant colorectal cancer.

Safety assessments were performed for all subjects after a first dosing of the investigational drug. Blood samples for PK and immunogenicity evaluation were collected after a single dosing and during a continuous dosing. Additionally, tumor imaging assessments were performed according to the Response Evaluation Criteria in Solid Tumors (RECIST v1.1).

2. Subject selection

Subjects had to meet all of the following inclusion criteria to be enrolled in this study: aged 18 years or older, regardless of gender; pathologically confirmed advanced solid tumor; failure of or intolerance to standard therapy, absence of standard therapy, or refusal of standard therapy; presence of an EGFR activating mutation, moderate to high expression, or amplification; and at least one measurable lesion confirmed by CT or MRI, according to the Response Evaluation Criteria in Solid Tumors (RECIST v1.1).

3. Study on the intervention and treatment

3.1 Investigational drug (DAR value determined using hydrophobic chromatography)

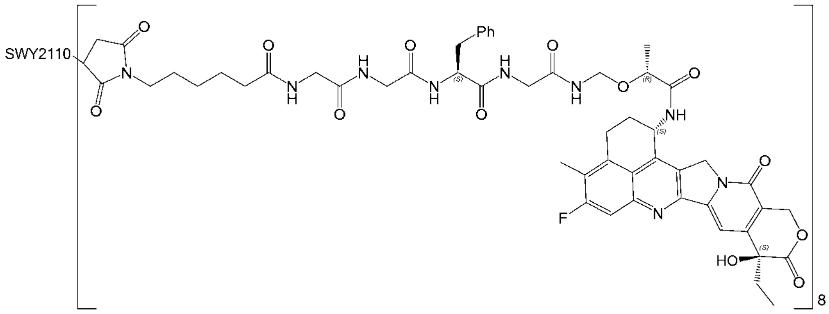

Dosage form: Lyophilized powder for injection;
Specification: 100 mg/vial;
Storage conditions: 2-8°C, protected from light.

3.2 Study on the implementation of the intervention

The initial dose of the investigational drug was calculated based on the subject's body weight at the screening stage/baseline. During the study of treatment, the dose did not need to be recalculated if the subject's body weight change was less than 10% compared to the screening stage/baseline. The medication was delivered via intravenous infusion once every three weeks (Q3W), with 21 days as one treatment cycle. The infusion duration was 60 min ± 10 min for the first dosing. If no infusion-related reaction occurred after the first dosing, the duration for each subsequent infusion was shortened to 30 min ± 5 min. The dosing continued until the treatment discontinuation criteria specified in the protocol were met. In the event of a delayed dosing, the dosing time for the subsequent cycle was calculated based on the actual date of the previous cycle's dosing.

4. Evaluation criteria

4.1 Pharmacokinetic evaluation

PK blood samples were collected from subjects at the planned sampling time-points and time-windows listed in Table 20 for different study stages to perform PK detection of the investigational drug. The detection components included toxin-conjugated antibodies, total antibodies, and free toxins.

For the dose escalation and PK expansion studies, a non-compartmental model was used to calculate PK parameters for each detection component of each subject, including: Cmax, Tmax, $t_{1/2}$, MRT, Vd, CL, $AUC_{0-t}$, $AUC_{0-\infty}$, $C_{max,ss}$, $C_{min,ss}$, $T_{max,ss}$, $AUC_{ss}$, and Rac. The pharmacokinetic parameters were descriptively summarized and tabulated. Box plots were created for the main dose-normalized PK parameters (such as AUC and $C_{max}$). An analysis of variance (ANOVA) model and/or a Power model were used to explore the relationship between main PK parameters and dose.

4.2 Immunogenicity evaluation

Immunogenicity blood samples were collected from subjects at the planned sampling time-points and time-windows listed in Table 20 for different study stages to perform the immunogenicity detection of the investigational drug. Immunogenicity samples were used for in-study cutpoint determination, anti-drug antibody (ADA) analysis, and target interference investigation.

The results for all anti-drug antibodies (ADAs) were tabulated. The proportion of ADA-positive subjects, the time to first ADA positivity, and the duration of positivity in subjects were analyzed. ADA-confirmed positive samples were further detected for neutralizing antibodies if necessary, and the results were tabulated. The impact of immunogenicity on drug PK, safety, and efficacy was analyzed.

Table 20: Collection of samples for pharmacokinetics and immunogenicity

| PK and Immunogenicity Sampling Time Points | | | Stage I | | Stage II | |
|---|---|---|---|---|---|---|
| Cycle/Day | Time | | PK | Immunogenicity | PK | Immunogenicity |
| | Before dosing | within 30 min | X | X | X | X |

(continued)

| PK and Immunogenicity Sampling Time Points | | | Stage I | | Stage II | |
|---|---|---|---|---|---|---|
| Cycle/Day | Time | | PK | Immunogenicity | PK | Immunogenicity |
| C1D1 | Immediately after dosing | +5 min | X | | X | |
| | After dosing | 2 h ± 10 min | X | | | |
| | | 6 h ± 10 min | X | | | |
| C1D2 | | 24 h ± 1 h | X | | | |
| C1D4 | | 72h ± 2 h | X | | | |
| C1D6 | | 120 h ± 4 h | X | | | |
| C1D8 | | 168 h ± 12 h | X | X | | |
| C1D15 | | 336 h ± 24 h | X | X | | |
| C2D1 | Before dosing | within 30 min | X | X | X | X |
| | Immediately after dosing | +5 min | X | | X | |
| C3D1 | Before dosing | within 30 min | X | X | X | X |
| | Immediately after dosing | +5 min | X | | X | |
| | After dosing | 2 h ± 10 min | X | | | |
| | | 6 h ± 10 min | X | | | |
| C3D2 | | 24 h ± 1 h | X | | | |
| C3D4 | | 72h ± 2 h | X | | | |
| C3D6 | | 120 h ± 4 h | X | | | |
| C3D8 | | 168 h ± 12 h | X | | | |
| C3D15 | | 336 h ± 24 h | X | | | |
| CnD1 (n=4, 6, 8) | Before dosing | within 30 min | X | X | X | X |
| | Immediately after dosing | +5 min | X | | X | |
| on D1 from Cycle 11 onwards, every 3 cycles (C11, C14...) | Before dosing | within 30 min | X | X | X | X |
| End of treatment and/or 30±7 days after the last dosing[1] | | | X | X | X | X |
| 60±7 days after the last dosing (if applicable) | | | | X | | X |
| 90±7 days after the last dosing (if applicable) | | | | X | | X |

(continued)

| PK and Immunogenicity Sampling Time Points | | Stage I | | Stage II | |
|---|---|---|---|---|---|
| Cycle/Day | Time | PK | Immunogenicity | PK | Immunogenicity |
| Within 72 hours after the occurrence of DLT or drug-related SAE (if applicable) | | X | X | X | X |
| 1: If the interval between the end of treatment and the 30-day visit after the last dosing was within 14 days, samples for PK and immunogenicity were only collected once. | | | | | |

4.3 Biomarker analysis

Tumor tissues or peripheral blood samples of the subjects were tested for EGFR status.

For EGFR-mutant Nsq-NSCLC, tumor tissue samples were also provided for IHC testing to analyze the correlation between EGFR expression levels and the efficacy of the investigational drug; tumor tissues or peripheral blood samples were provided during NSCLC screening for NGS testing to analyze factors affecting the efficacy of the investigational drug.

4.4 Safety evaluation

Adverse events were recorded and graded for severity using the "U.S. National Cancer Institute Common Terminology Criteria for Adverse Events" (NCI-CTCAE, version 5.0). Adverse events were collected and evaluated to survey the causal relationship between adverse events and the drug.

Any adverse events (AEs) in all subjects, including clinical symptoms, abnormal vital signs, and any abnormalities detected in laboratory tests, were observed and recorded, and their relationship to the investigational drug was assessed.

For subjects in the dose escalation study, DLT events were observed during the DLT observation period; delayed DLTs were observed within 84 days after the first dosing.

It was necessary for the subjects to perform safety evaluations at the screening stage/baseline and after dosing, including physical examination, ECOG scoring, laboratory tests, and electrocardiogram examination.

Skin toxicity, interstitial pneumonia/non-infectious pneumonia toxicity, hematological toxicity, gastrointestinal toxicity, ocular toxicity, hepatotoxicity, infusion-related reactions /allergic reactions, and other toxicities were investigated and assessed.

The occurrence of any skin adverse events in the subjects was observed, and the skin adverse events were graded according to CTCAE v5.0 and managed.

Subjects were monitored for radiographic changes consistent with interstitial lung disease/ non-infectious pneumonia, or acute onset, or new or worsening pulmonary or other related signs/symptoms, such as dyspnea, cough, or fever, and assessed for the occurrence of interstitial lung disease/ non-infectious pneumonia. Pulmonary function tests, including assessment of key parameters related to ventilation and gas exchange, were performed at the screening stage.

Complete blood counts of the subjects were monitored regularly.

Subjects were observed for symptoms such as diarrhea, loose stools, vomiting, and nausea.

Subjects were observed for ocular toxicity, such as acute or worsening eye inflammation, tearing, photosensitivity, blurred vision, eye pain, and/or eye redness, and the adverse events were classified according to CTCAE V5.0. An ophthalmic examination, the items of which included visual acuity, slit-lamp examination, and fundus examination, was performed at the screening stage.

Subjects were monitored for abnormal liver function.

Subjects were observed for infusion-related reaction/allergic reaction, such as fever, pruritus, rash, angioedema, chills, hypotension, dyspnea, chest discomfort, wheezing or tachycardia, and anaphylaxis, and the adverse events were classified according to CTCAE V5.0.

4.5 Efficacy evaluation

Efficacy was evaluated using the RECIST 1.1 standard with CT/MRI imaging assessments. Subjects underwent tumor imaging assessments every 6 weeks within the first 24 weeks after the first dosing and then every 9 weeks after 24 weeks. A time window of ±7 days was allowed for imaging examinations. Efficacy evaluations were performed at the scheduled timepoints and were not delayed due to the delay of dosing.

Efficacy endpoints included:

Objective response rate (ORR): Defined as the proportion of subjects with a best overall response of complete response

(CR) or partial response (PR) (i.e., CR+PR) in the study, as assessed by the investigator according to RECIST v1.1 during the period from the start of the investigational drug until the drop-out from the study.

Disease control rate (DCR): Defined as the proportion of subjects with a best timepoint response of CR, PR, or stable disease (SD) (i.e., CR+PR+SD) assessed according to RECIST v1.1 guidelines during the period from the start of the investigational drug until the drop-out from the study.

Progression-free survival (PFS): Defined as the time from the start of the investigational drug to the first record of disease progression (PD) or death from any cause, whichever occurs first.

Duration of response (DoR): Defined as the time from the first tumor assessment of CR or PR to the first assessment of PD or death from any cause, whichever occurs first.

Overall survival (OS): Defined as the time from the start of the investigational drug to death from any cause.

Efficacy was descriptively summarized by dose group in the first stage. In the second stage, the best overall response (BOR), ORR, and DCR were summarized and analyzed. PFS, DoR, and OS were also analyzed. The analysis methods were outlined as follows: □

BOR was summarized by cohort according to RECIST 1.1, and the number and percentage of ORR and DCR were calculated, with 95% confidence intervals (CI) calculated using the Clopper-Pearson exact method.

□ Kaplan-Meier analysis was used for PFS, DoR, and OS, which included estimating the median time and its 95% CI, plotting Kaplan-Meier curves, and the like.

5. Study results

A total of 25 subjects were enrolled, and safety data were available for 23 subjects. A total of 16 subjects completed at least one efficacy evaluation. There were 4 PRs, 11 SDs, and 1 PD.

0.6 mg/kg dose group: 1 SD (EGFR mutant, non-squamous NSCLC)

1.8 mg/kg dose group: 3 SDs (two subjects of EGFR mutant non-squamous NSCLC and one colorectal cancer with moderate/high EGFR expression)

3.6 mg/kg dose group: 2 SDs (one subject of lung squamous carcinoma with moderate/high EGFR expression and one non-squamous NSCLC with moderate/high EGFR expression), and 1 PD (colorectal cancer with moderate/high EGFR expression, RAS/BRAF wild type);

4.8 mg/kg dose group: 3 PRs (one subject of EGFR mutated/amplified non-squamous NSCLC; one subject of lung squamous carcinoma with EGFR moderate to high expression/mutation; one subject of nasopharyngeal carcinoma with moderate/high EGFR expression), 3 SDs (two subjects of non-squamous NSCLC with EGFR mutation, one subject of EGFR mutated/amplified non-squamous NSCLC)

6.4 mg/kg dose group: 1 PR (EGFR mutated/amplified non-squamous NSCLC), 2 SDs (one subject of lung squamous carcinoma with moderate/high EGFR expression, and one subject of non-squamous NSCLC with moderate/high EGFR expression)

13 subjects of non-small cell lung cancer: 3 PRs and 10 SDs; one subject of nasopharyngeal carcinoma: PR; and two subjects of colorectal cancer: 1 SD and 1 PD.

Typical cases

Case 1: A 69-year-old male was initially diagnosed with non-small cell lung cancer. He underwent a surgical treatment (left upper lobectomy+mediastinal lymphadenectomy) followed by 4 cycles of vinorelbine plus cisplatin, but the disease progressed. Subsequently, he started an oral osimertinib treatment, followed by a treatment of paclitaxel + carboplatin combined with osimertinib, but the disease progressed. He then received radiotherapy for bone metastases. Subsequently, he received an afatinib treatment, but the disease progressed. He then started a treatment of paclitaxel combined with cisplatin, but the disease progressed, followed by a treatment of paclitaxel + cisplatin combined with tislelizumab, but the disease progressed.

An informed consent form was then signed. The patient's clinical stage at the screening stage was IV, with moderate/high expression of EGFR and T790M mutation. At baseline, the sum of the target lesions was 168 mm, including the largest lesion in the lung measuring 84 mm and new cavitation. The first efficacy assessment after receiving the investigational drug resulted in a PR.

Case 2: A 56-year-old male was diagnosed with non-small cell lung cancer. He was treated with JNJ-61186372 combined with Lazertinib. After disease progression, he participated in another clinical trial. After a further disease progression, he received a treatment of pemetrexed combined with carboplatin, but discontinued chemotherapy due to myelosuppression.

An informed consent form was then signed. The patient's clinical stage at the screening stage was IV, with EGFR

mutation and amplification. The first efficacy assessment after receiving the investigational drug resulted in a PR, which was confirmed at the second efficacy assessment.

Case 3: A 48-year-old female was diagnosed with non-small cell lung cancer. She participated in another drug trial, but the disease progressed.

An informed consent form was then signed. The patient's clinical stage at the screening stage was IV, with EGFR mutation and amplification. The first efficacy assessment after receiving the investigational drug resulted in a PR.

Case 4: A 55-year-old male was initially diagnosed with nasopharyngeal carcinoma. He was treated with gemcitabine + cisplatin, concurrent chemoradiotherapy, followed by capecitabine maintenance therapy, but the disease progressed; then treated with camrelizumab, but the disease progressed; then treated with docetaxel + nedaplatin, but the disease progressed.

An informed consent form was then signed. The patient's clinical stage at the screening stage was IV, with moderate/high EGFR expression. The first efficacy assessment after receiving the investigational drug resulted in a PR.

The clinical study demonstrated that the antibody-drug conjugate represented by Formula I, or a pharmaceutically acceptable salt, hydrate, solvate, stereoisomer, or isotopic label thereof could effectively treat advanced malignant tumors, particularly EGFR-positive advanced solid tumors; could overcome resistance to EGFR-TKIs and EGFR monoclonal antibodies; could provide clinical benefits superior to existing treatments for patients with advanced solid tumors, including non-small cell lung cancer; and could control or alleviate the disease.

## Claims

1. An antibody-drug conjugate represented by Formula I, or a pharmaceutically acceptable salt, hydrate, solvate, stereoisomer, or isotopic label thereof for use in treating a tumor:

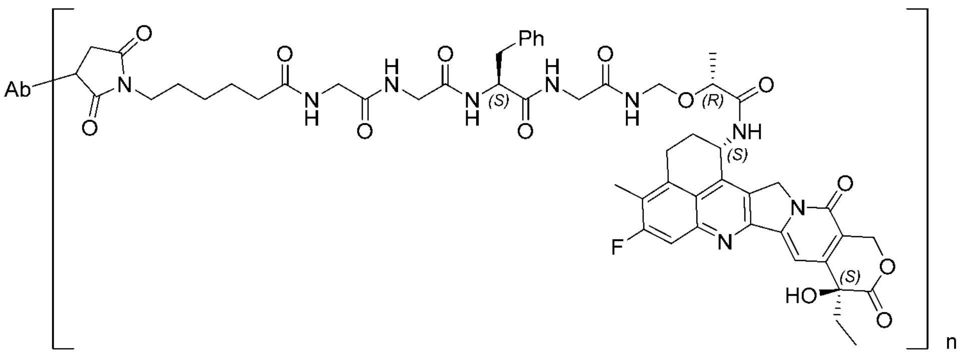

(I)

wherein Ab is selected from SWY2110, SWY2111, SWY2112, and SWY2113;

SWY2110 is an antibody having two heavy chains with the amino acid sequence shown in SEQ ID NO:29 and two light chains with the amino acid sequence shown in SEQ ID NO:30;
SWY2111 is an antibody having two heavy chains with the amino acid sequence shown in SEQ ID NO:34 and two light chains with the amino acid sequence shown in SEQ ID NO:30;
SWY2112 is an antibody having two heavy chains with the amino acid sequence shown in SEQ ID NO:37 and two light chains with the amino acid sequence shown in SEQ ID NO:30;
SWY2113 is an antibody having two heavy chains with the amino acid sequence shown in SEQ ID NO:40 and two light chains with the amino acid sequence shown in SEQ ID NO:30;
n is an integer of 1-8 or a decimal of 1-8; preferably, n is a number between 7 and 8, inclusive of the endpoints 7 and 8;
the tumor is selected from triple-negative breast cancer, gastric cancer, esophageal cancer, pancreatic cancer, cervical cancer, biliary tract cancer, HR-positive breast cancer, liver cancer, ovarian cancer, endometrial cancer, prostate cancer, lung squamous carcinoma, non-squamous non-small cell lung cancer, head and neck squamous carcinoma (including nasopharyngeal carcinoma), colorectal cancer, urothelial carcinoma, kidney cancer, and the like, preferably, the tumor is selected from EGFR moderate/high-expression colorectal cancer; EGFR moderate/high-expression triple-negative breast cancer; EGFR moderate/high-expression head and neck squamous carcinoma (including nasopharyngeal carcinoma); EGFR mutant non-squamous non-small cell lung cancer (Nsq-NSCLC); lung squamous carcinoma with moderate/high EGFR expression (Sq-NSCLC); other advanced solid tumors with moderate/high EGFR expression such as gastric cancer, esophageal cancer,

pancreatic cancer, cervical cancer, biliary tract cancer, non-squamous non-small cell lung cancer, and RAS/-BRAF mutant colorectal cancer; drug-resistant lung cancer (such as Gefitinib and/or Osimertinib-resistant lung cancer).

2. The antibody-drug conjugate represented by Formula I, or a pharmaceutically acceptable salt, hydrate, solvate, stereoisomer, or isotopic label thereof for use in treating a tumor according to claim 1, **characterized in that**: each administration amount calculated as the antibody-drug conjugate represented by Formula I is: 1-1000 mg/dosing, preferably 5-500 mg/dosing, or 10-300 mg/dosing, or 20-200 mg/dosing, or 50-150 mg/dosing, or 50-300 mg/dosing, or 50-400 mg/dosing, or 100-200 mg/dosing, or 100-300 mg/dosing, or 100-500 mg/dosing, more preferably 5 mg/dosing, 6 mg/dosing, 8 mg/dosing, 10 mg/dosing, 15 mg/dosing, 20 mg/dosing, 25 mg/dosing, 30 mg/dosing, 40 mg/dosing, 50 mg/dosing, 60 mg/dosing, 70 mg/dosing, 80 mg/dosing, 90 mg/dosing, 100 mg/dosing, 150 mg/dosing, 200 mg/dosing, 250 mg/dosing, 300 mg/dosing, 500 mg/dosing, 600 mg/dosing, 700 mg/dosing, 800 mg/dosing, 900 mg/dosing, or the like.

3. The antibody-drug conjugate represented by Formula I, or a pharmaceutically acceptable salt, hydrate, solvate, stereoisomer, or isotopic label thereof for use in treating a tumor according to claim 1, **characterized in that**: each administration amount calculated as the antibody-drug conjugate represented by Formula I and the body weight of patient is: 0.05-50 mg/kg, preferably 0.1-20 mg/kg, or 0.2-20 mg/kg, or 0.5-20 mg/kg, or 0.5-15 mg/kg, or 0.5-10 mg/kg, or 0.5-8 mg/kg, or 0.5-6 mg/kg, or 0.5-4 mg/kg, or 0.5-2 mg/kg, or 1-20 mg/kg, or 1-10 mg/kg, or 1-8 mg/kg, or 2-20 mg/kg, or 2-10 mg/kg, or 2-8 mg/kg, or 3-20 mg/kg, or 3-10 mg/kg, or 3-8 mg/kg, or 5-20 mg/kg, or 5-10 mg/kg, or 0.3 mg/kg, or 0.4 mg/kg, or 0.6 mg/kg, or 0.8 mg/kg, or 1.5 mg/kg, or 1.8 mg/kg, or 2.0 mg/kg, or 2.2 mg/kg, or 2.4 mg/kg, or 2.5 mg/kg, or 2.8 mg/kg, or 3 mg/kg, or 3.2 mg/kg, or 3.5 mg/kg, or 3.6 mg/kg, or 3.8 mg/kg, or 4.0 mg/kg, or 4.2 mg/kg, or 4.4 mg/kg, or 4.5 mg/kg, or 4.6 mg/kg, or 4.8 mg/kg, or 5.0 mg/kg, or 5.2 mg/kg, or 5.4 mg/kg, or 5.5 mg/kg, or 5.6 mg/kg, or 5.8 mg/kg, or 6.0 mg/kg, or 6.2 mg/kg, or 6.4 mg/kg, or 6.8 mg/kg, or 7.0 mg/kg, or 7.2 mg/kg, or 7.5 mg/kg, or 7.8 mg/kg, or 8.0 mg/kg, or 8.5 mg/kg, or 9.0 mg/kg, or 9.5 mg/kg, or 11 mg/kg, or 11.5 mg/kg, or 12.0 mg/kg, or 16.8 mg/kg, or 18.0mg/kg.

4. The antibody-drug conjugate represented by Formula I, or a pharmaceutically acceptable salt, hydrate, solvate, stereoisomer, or isotopic label thereof for use in treating a tumor according to claim 1, **characterized in that**: the antibody-drug conjugate represented by Formula I, or a pharmaceutically acceptable salt, hydrate, solvate, stereoisomer, or isotopic label thereof can be administered once daily (QD) or can be administered by dividing the therapeutically effective amount into multiple administrations within one day, for example, twice daily (BID) or three times daily (TID); alternatively can be administered once weekly (QW) or can be administered by dividing the aforementioned therapeutically effective amount as the total weekly dosage into multiple-day administrations within a week, for example, twice weekly (BIW) or thrice weekly (TIW); alternatively can be administered once every two weeks (Q2W), or can be administered by dividing into multiple-day administrations within two weeks, for example, twice every two weeks (BI2W) or three times every two weeks (TI2W); alternatively can be administered once every three weeks (Q3W), or can be administered by dividing into multiple-day administrations within three weeks, for example, twice every three weeks (BI3W, e.g. D1 and D8 in one cycle every three weeks) or three times every three weeks (TI3W); alternatively can be administered once monthly (QM), or can be administered by dividing into multiple-day administrations within one month, for example, twice monthly (BIM) or three times monthly (TIM); alternatively can be administered at intervals e.g. administrated once at an interval of 2-7 days, or administrated once at an interval of 3 days, or 4 days, or 5 days, or 6 days, or administrated once at an interval of 2-4 weeks, e.g. administrated once at an interval of 2 weeks (administrated once every 3 weeks with a cycle of every 3 weeks), administrated once at an interval of 1 week (once every 2 weeks with a cycle of every 2 weeks or a cycle of every 4 weeks) and administrated once at an interval of 3 weeks (once every 4 weeks with a cycle of every 4 weeks), or administrated once at an interval of 1-3 months, e.g. administrated once at an interval of 1 month (every 2 months); or continuously administered for 3 weeks and then stopped for 1 week with a cycle of every 4 weeks, or continuously administered for 2 weeks and then stopped for 1 week with a cycle of every 3 weeks, or continuously administered for 2 weeks and then stopped for 2 weeks with a cycle of every 4 weeks, or administered at an interval of 1 week (once every 2 weeks) with a cycle of every 4 weeks; or administered once on Day 1 in each cycle with a cycle of every 3 weeks.

5. The antibody-drug conjugate represented by Formula I, or a pharmaceutically acceptable salt, hydrate, solvate, stereoisomer, or isotopic label thereof for use in treating a tumor according to claim 4, **characterized in that** the antibody-drug conjugate represented by Formula I, or a pharmaceutically acceptable salt, hydrate, solvate, stereoisomer, or isotopic label thereof, is administered at an administration amount according to claim 2 or 3 in each administration cycle.

6. Use of an antibody-drug conjugate represented by Formula I, or a pharmaceutically acceptable salt, hydrate, solvate, stereoisomer, or isotopic label thereof:

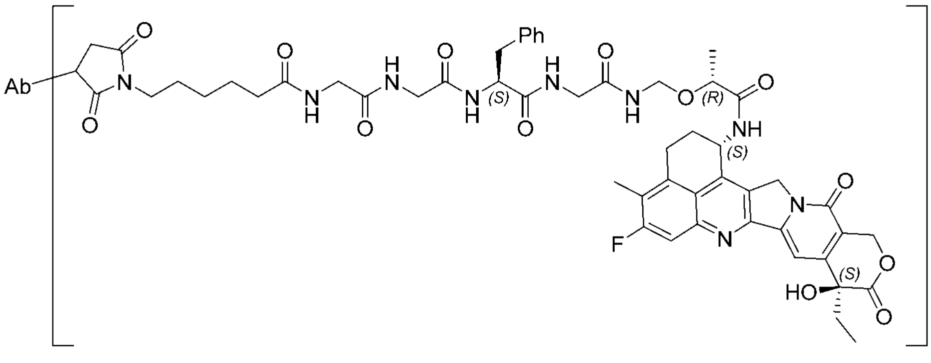

n (I)

wherein Ab is selected from SWY2110, SWY2111, SWY2112, and SWY2113;

SWY2110 is an antibody having two heavy chains with the amino acid sequence shown in SEQ ID NO:29 and two light chains with the amino acid sequence shown in SEQ ID NO:30;
SWY2111 is an antibody having two heavy chains with the amino acid sequence shown in SEQ ID NO:34 and two light chains with the amino acid sequence shown in SEQ ID NO:30;
SWY2112 is an antibody having two heavy chains with the amino acid sequence shown in SEQ ID NO:37 and two light chains with the amino acid sequence shown in SEQ ID NO:30;
SWY2113 is an antibody having two heavy chains with the amino acid sequence shown in SEQ ID NO:40 and two light chains with the amino acid sequence shown in SEQ ID NO:30;
n is an integer of 1-8 or a decimal of 1-8; preferably, n is a number between 7 and 8, inclusive of the endpoints 7 and 8;
in manufacture of a medicament for treating a tumor, wherein the tumor is selected from triple-negative breast cancer, gastric cancer, esophageal cancer, pancreatic cancer, cervical cancer, biliary tract cancer, HR-positive breast cancer, liver cancer, ovarian cancer, endometrial cancer, prostate cancer, lung squamous carcinoma, non-squamous non-small cell lung cancer, head and neck squamous carcinoma (including nasopharyngeal carcinoma), colorectal cancer, urothelial carcinoma, kidney cancer, and the like, preferably, the tumor is selected from EGFR moderate/high-expression colorectal cancer; EGFR moderate/high-expression triple-negative breast cancer; EGFR moderate/high-expression head and neck squamous carcinoma (including nasopharyngeal carcinoma); EGFR mutant non-squamous non-small cell lung cancer (Nsq-NSCLC); lung squamous carcinoma with moderate/high EGFR expression (Sq-NSCLC); other advanced solid tumors with moderate/high EGFR expression such as gastric cancer, esophageal cancer, pancreatic cancer, cervical cancer, biliary tract cancer, non-squamous non-small cell lung cancer, and RAS/BRAF mutant colorectal cancer; drug-resistant lung cancer (such as Gefitinib and/or Osimertinib-resistant lung cancer).

7. A pharmaceutical preparation containing an antibody-drug conjugate represented by Formula I, or a pharmaceutically acceptable salt, hydrate, solvate, stereoisomer, or isotopic label thereof:

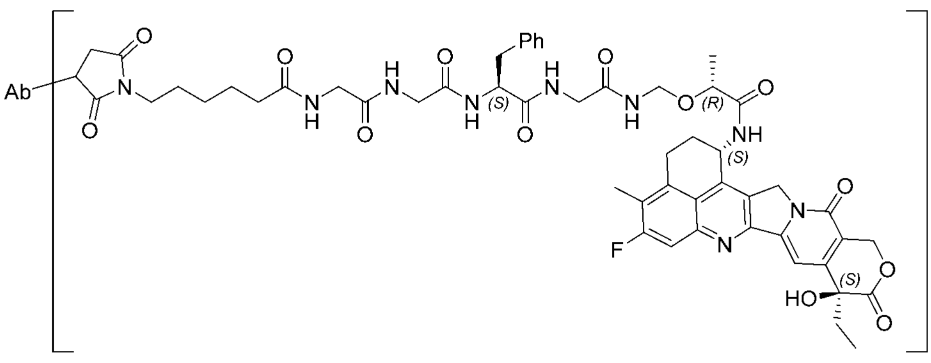

n (I)

wherein Ab is selected from SWY2110, SWY2111, SWY2112, and SWY2113;

SWY2110 is an antibody having two heavy chains with the amino acid sequence shown in SEQ ID NO:29 and two light chains with the amino acid sequence shown in SEQ ID NO:30;
SWY2111 is an antibody having two heavy chains with the amino acid sequence shown in SEQ ID NO:34 and two light chains with the amino acid sequence shown in SEQ ID NO:30;
SWY2112 is an antibody having two heavy chains with the amino acid sequence shown in SEQ ID NO:37 and two light chains with the amino acid sequence shown in SEQ ID NO:30;
SWY2113 is an antibody having two heavy chains with the amino acid sequence shown in SEQ ID NO:40 and two light chains with the amino acid sequence shown in SEQ ID NO:30;
n is an integer of 1-8 or a decimal of 1-8; preferably, n is a number between 7 and 8, inclusive of the endpoints 7 and 8;
said pharmaceutical preparation is an oral preparation (such as tablet and capsule) or an injection preparation (such as injection solution and lyophilized powder for injection).

**8.** The pharmaceutical preparation according to claim 7, **characterized in that** each dosing unit of the pharmaceutical preparation contains the antibody-drug conjugate represented by Formula I, or a pharmaceutically acceptable salt, hydrate, solvate, stereoisomer, or isotopic label thereof, calculated as the antibody-drug conjugate represented by Formula I, in an amount of 1-1000mg, preferably 5-500mg, or 10-300mg, or 20-200mg, or 50-150mg, or 50-300mg, or 50-400mg, or 100-200mg, or 100-300mg, or 100-500mg, more preferably 1mg, 5mg, 10mg, 20mg, 30mg, 40mg, 50mg, 60mg, 70mg, 80mg, 90mg, 100mg, 150mg, 200mg, 250mg, 300mg, 500mg, 1000mg, or the like.

**9.** A method of treating a tumor, **characterized in that** the antibody-drug conjugate represented by Formula I, or a pharmaceutically acceptable salt, hydrate, solvate, stereoisomer, or isotopic label thereof, is administered at a therapeutically effective amount to a subject or patient in need of treatment to treat tumors:

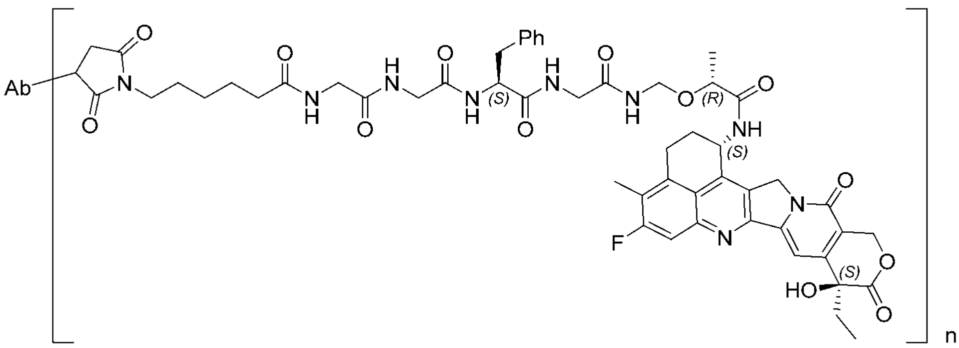
(I)

wherein Ab is selected from SWY2110, SWY2111, SWY2112, and SWY2113;

SWY2110 is an antibody having two heavy chains with the amino acid sequence shown in SEQ ID NO:29 and two light chains with the amino acid sequence shown in SEQ ID NO:30;
SWY2111 is an antibody having two heavy chains with the amino acid sequence shown in SEQ ID NO:34 and two light chains with the amino acid sequence shown in SEQ ID NO:30;
SWY2112 is an antibody having two heavy chains with the amino acid sequence shown in SEQ ID NO:37 and two light chains with the amino acid sequence shown in SEQ ID NO:30;
SWY2113 is an antibody having two heavy chains with the amino acid sequence shown in SEQ ID NO:40 and two light chains with the amino acid sequence shown in SEQ ID NO:30;
n is an integer of 1-8 or a decimal of 1-8; preferably, n is a number between 7 and 8, inclusive of the endpoints 7 and 8;
the therapeutically effective amount refers to a dose sufficient to alleviate or inhibit the tumor growth, or eliminate the tumor, benefiting the subject or patient, without causing intolerable toxic side effects, the tumor is selected from triple-negative breast cancer, gastric cancer, esophageal cancer, pancreatic cancer, cervical cancer, biliary tract cancer, HR-positive breast cancer, liver cancer, ovarian cancer, endometrial cancer, prostate cancer, lung squamous carcinoma, non-squamous non-small cell lung cancer, head and neck squamous carcinoma (including nasopharyngeal carcinoma), colorectal cancer, urothelial carcinoma, kidney cancer, and the like, preferably, the tumor is selected from EGFR moderate/high-expression colorectal cancer; EGFR moderate/high-expression triple-negative breast cancer; EGFR moderate/high-expression head and neck squamous carcinoma (including nasopharyngeal carcinoma); EGFR mutant non-squamous non-small cell lung cancer (Nsq-NSCLC); lung

squamous carcinoma with moderate/high EGFR expression (Sq-NSCLC); other advanced solid tumors with moderate/high EGFR expression such as gastric cancer, esophageal cancer, pancreatic cancer, cervical cancer, biliary tract cancer, non-squamous non-small cell lung cancer, and RAS/BRAF mutant colorectal cancer; drug-resistant lung cancer (such as Gefitinib and/or Osimertinib-resistant lung cancer).

**10.** The method according to claim 9, **characterized in that** each administration amount, calculated as the antibody-drug conjugate represented by Formula I, is 1-1000 mg/dosing, preferably 5-500 mg/dosing, or 10-300 mg/dosing, or 20-200 mg/dosing, or 50-150 mg/dosing, or 50-300 mg/dosing, or 50-400 mg/dosing, or 100-200 mg/dosing, or 100-300 mg/dosing, or 100-500 mg/dosing, more preferably 5 mg/dosing, 6 mg/dosing, 8 mg/dosing, 10 mg/dosing, 15 mg/dosing, 20 mg/dosing, 25 mg/dosing, 30 mg/dosing, 40 mg/dosing, 50 mg/dosing, 60 mg/dosing, 70 mg/dosing, 80 mg/dosing, 90 mg/dosing, 100 mg/dosing, 150 mg/dosing, 200 mg/dosing, 250 mg/dosing, 300 mg/dosing, 500 mg/dosing, 600 mg/dosing, 700 mg/dosing, 800 mg/dosing, 900 mg/dosing, or the like.

**11.** The method according to claim 9, **characterized in that** each administration amount, calculated as the antibody-drug conjugate represented by Formula I and the body weight of patient, is 0.05-50 mg/kg, preferably 0.1-20 mg/kg, or 0.2-20 mg/kg, or 0.5-20 mg/kg, or 0.5-15 mg/kg, or 0.5-10 mg/kg, or 0.5-8 mg/kg, or 0.5-6 mg/kg, or 0.5-4 mg/kg, or 0.5-2 mg/kg, or 1-20 mg/kg, or 1-10 mg/kg, or 1-8 mg/kg, or 2-20 mg/kg, or 2-10 mg/kg, or 2-8 mg/kg, or 3-20 mg/kg, or 3-10 mg/kg, or 3-8 mg/kg, or 5-20 mg/kg, or 5-10 mg/kg, or 0.3 mg/kg, or 0.4 mg/kg, or 0.6 mg/kg, or 0.8 mg/kg, or 1.5 mg/kg, or 1.8 mg/kg, or 2.0 mg/kg, or 2.2 mg/kg, or 2.4 mg/kg, or 2.5 mg/kg, or 2.8 mg/kg, or 3 mg/kg, or 3.2 mg/kg, or 3.5 mg/kg, or 3.6 mg/kg, or 3.8 mg/kg, or 4.0 mg/kg, or 4.2 mg/kg, or 4.4 mg/kg, or 4.5 mg/kg, or 4.6 mg/kg, or 4.8 mg/kg, or 5.0 mg/kg, or 5.2 mg/kg, or 5.4 mg/kg, or 5.5 mg/kg, or 5.6 mg/kg, or 5.8 mg/kg, or 6.0 mg/kg, or 6.2 mg/kg, or 6.4 mg/kg, or 6.8 mg/kg, or 7.0 mg/kg, or 7.2 mg/kg, or 7.5 mg/kg, or 7.8 mg/kg, or 8.0 mg/kg, or 8.5 mg/kg, or 9.0 mg/kg, or 9.5 mg/kg, or 11 mg/kg, or 11.5 mg/kg, or 12.0 mg/kg, or 16.8 mg/kg, or 18.0mg/kg.

**12.** The method according to claim 9, **characterized in that** the antibody-drug conjugate represented by Formula I, or a pharmaceutically acceptable salt, hydrate, solvate, stereoisomer, or isotopic label thereof can be administered once daily (QD) or can be administered by dividing the therapeutically effective amount into multiple administrations within one day, for example, twice daily (BID) or three times daily (TID); alternatively can be administered once weekly (QW) or can be administered by dividing the aforementioned therapeutically effective amount as the total weekly dosage into multiple-day administrations within a week, for example, twice weekly (BIW) or thrice weekly (TIW); alternatively can be administered once every two weeks (Q2W), or can be administered by dividing into multiple-day administrations within two weeks, for example, twice every two weeks (BI2W) or three times every two weeks (TI2W); alternatively can be administered once every three weeks (Q3W), or can be administered by dividing into multiple-day administrations within three weeks, for example, twice every three weeks (BI3W, e.g. D1 and D8 in one cycle every three weeks) or three times every three weeks (TI3W); alternatively can be administered once monthly (QM), or can be administered by dividing into multiple-day administrations within one month, for example, twice monthly (BIM) or three times monthly (TIM); alternatively can be administered at intervals e.g. administrated once at an interval of 2-7 days, or administrated once at an interval of 3 days, or 4 days, or 5 days, or 6 days, or administrated once at an interval of 2-4 weeks, e.g. administrated once at an interval of 2 weeks (administrated once every 3 weeks with a cycle of every 3 weeks), administrated once at an interval of 1 week (once every 2 weeks with a cycle of every 2 weeks or a cycle of every 4 weeks) and administrated once at an interval of 3 weeks (once every 4 weeks with a cycle of every 4 weeks), or administrated once at an interval of 1-3 months, e.g. administrated once at an interval of 1 month (every 2 months); or continuously administered for 3 weeks and then stopped for 1 week with a cycle of every 4 weeks, or continuously administered for 2 weeks and then stopped for 1 week with a cycle of every 3 weeks, or continuously administered for 2 weeks and then stopped for 2 weeks with a cycle of every 4 weeks, or administered at an interval of 1 week (once every 2 weeks) with a cycle of every 4 weeks; or administered once on Day 1 in each cycle with a cycle of every 3 weeks.

**13.** The method according to claim 12, **characterized in that** the antibody-drug conjugate represented by Formula I, or a pharmaceutically acceptable salt, hydrate, solvate, stereoisomer, or isotopic label thereof, is administered at an administration amount according to claim 10 or 11 in each administration cycle.

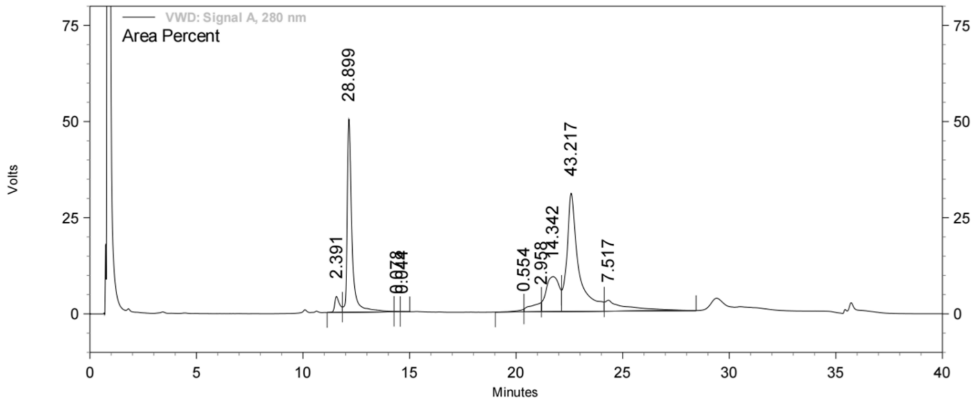


Figure 1

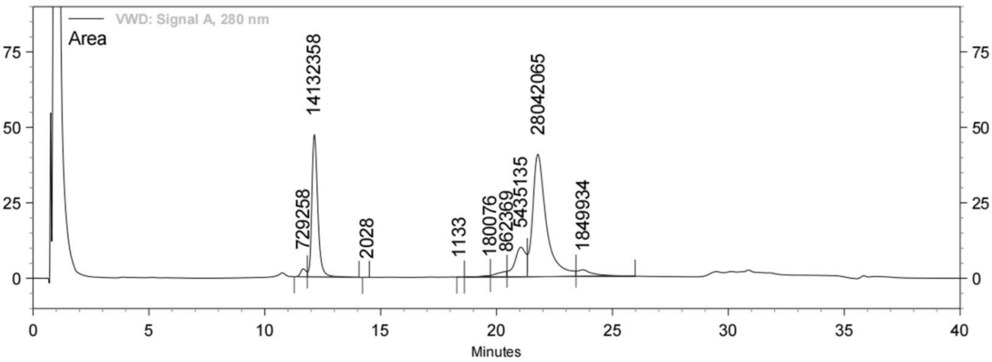


Figure 2

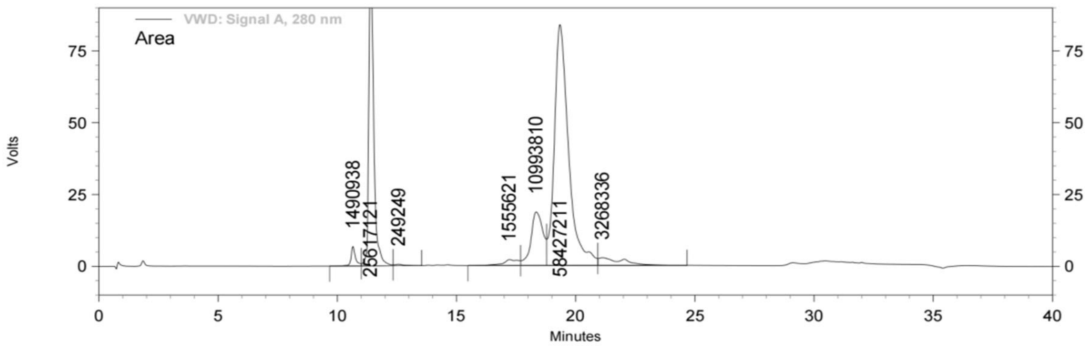


Figure 3

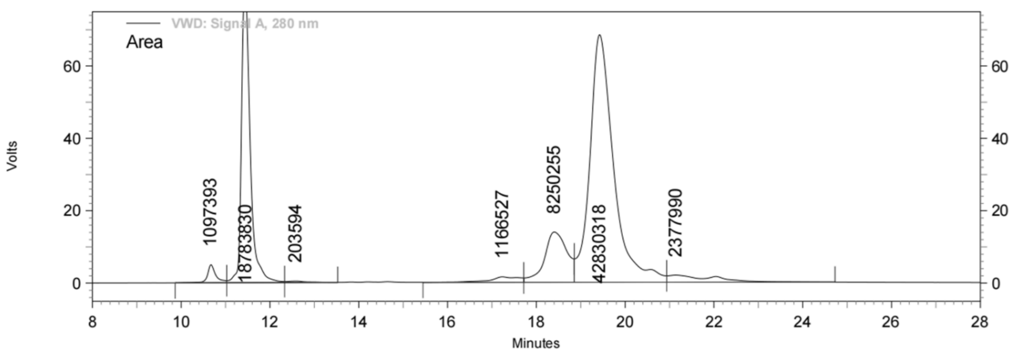


Figure 4

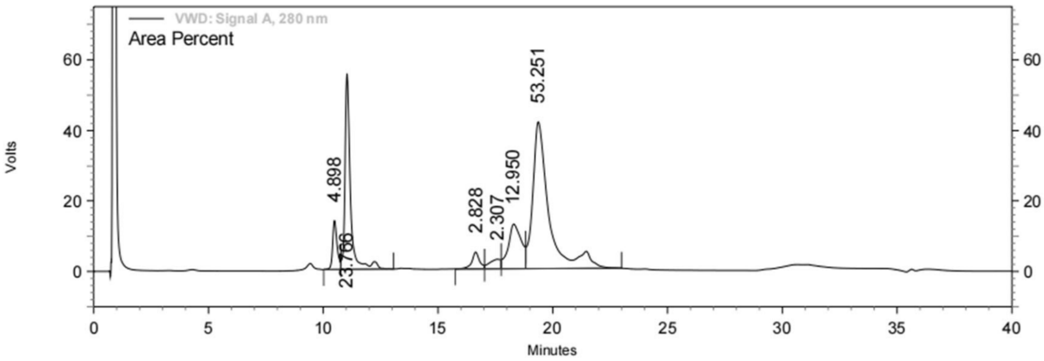


Figure 5

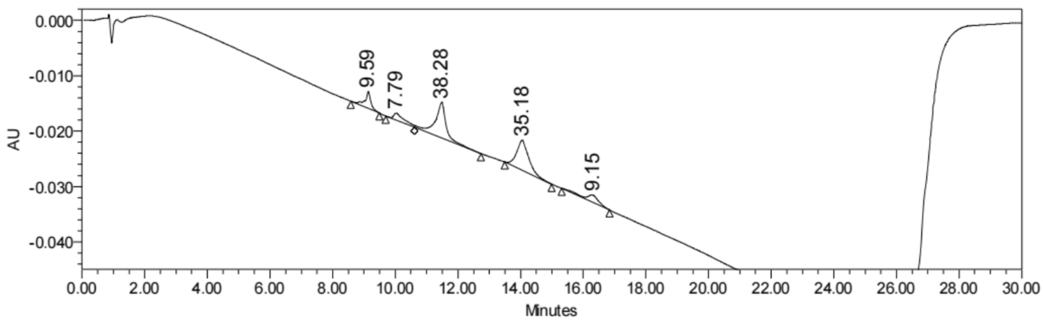


Figure 6

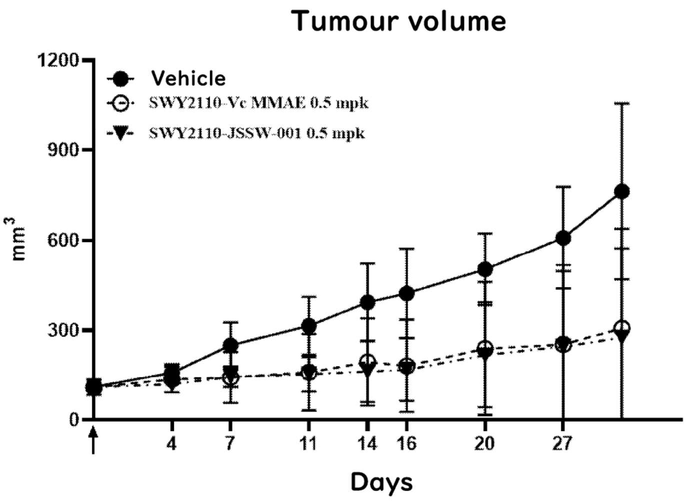


Figure 7

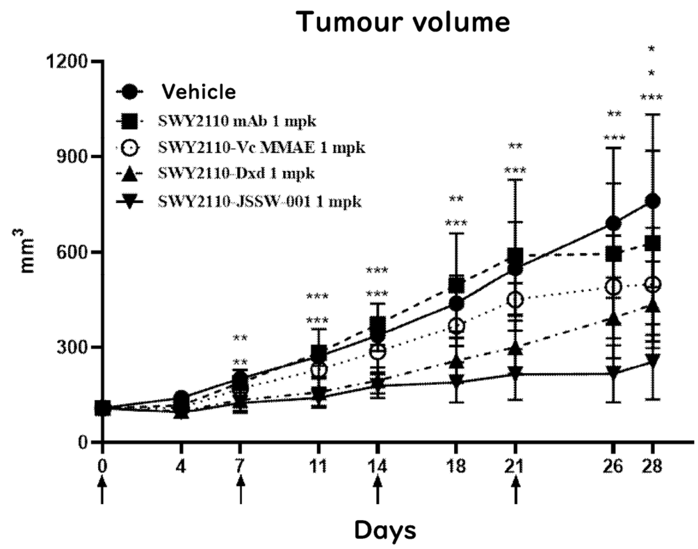


Figure 8

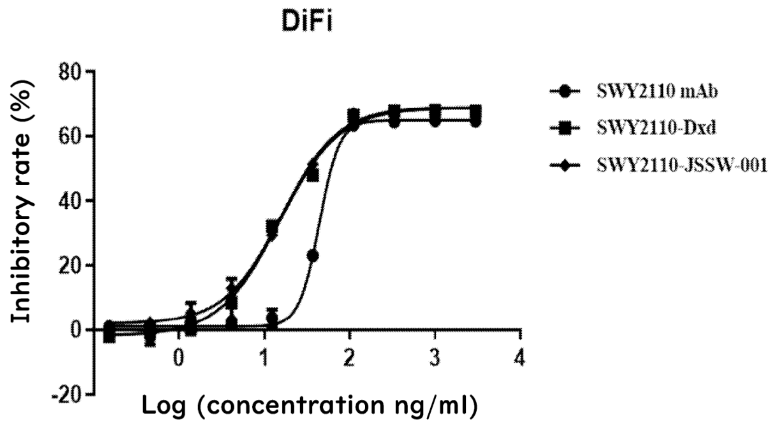


Figure 9

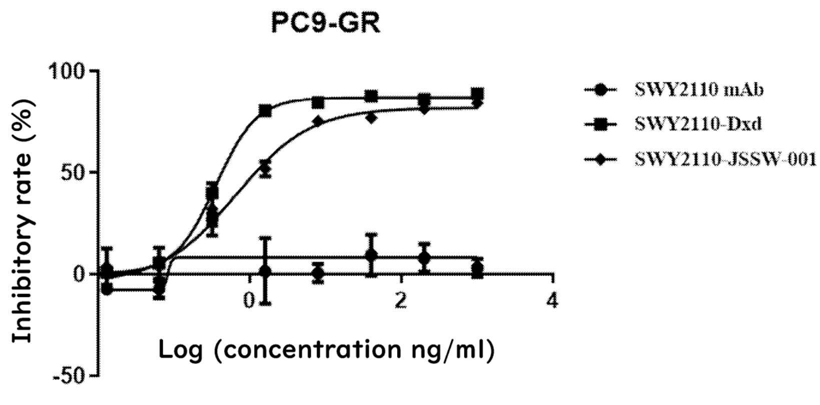


Figure 10

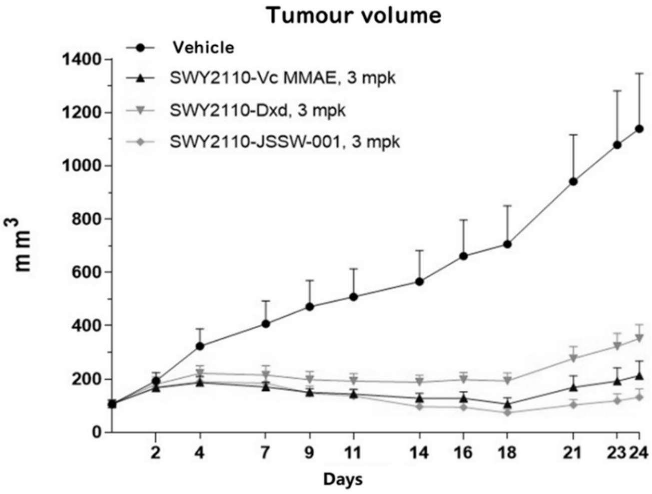


Figure 11

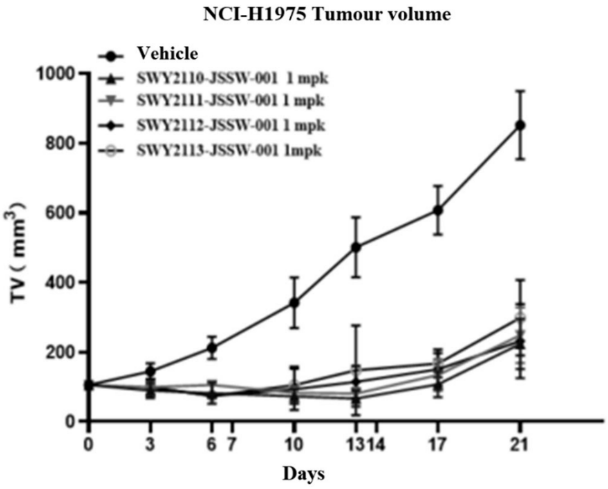


Figure 12

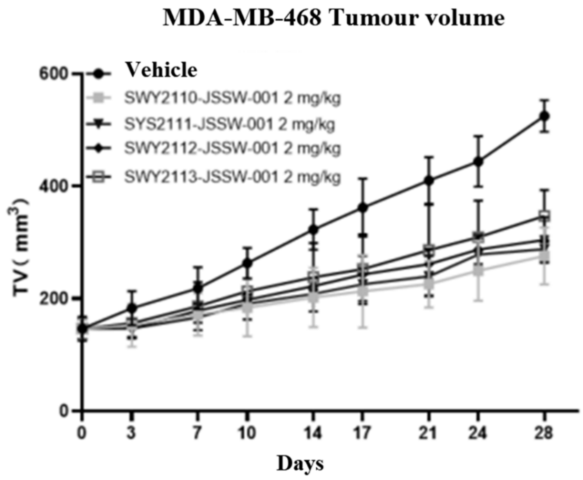


Figure 13

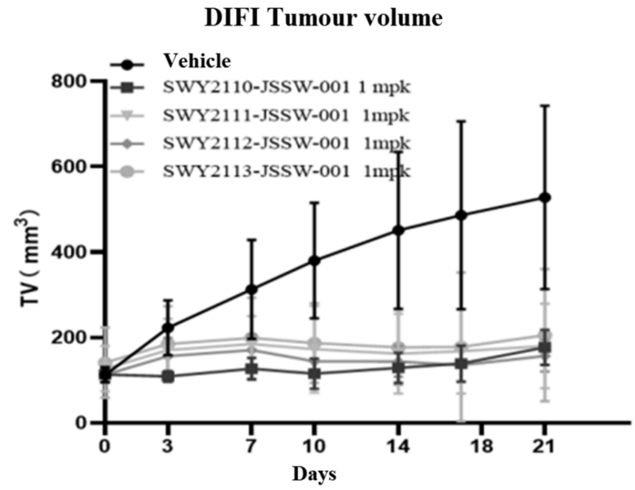


Figure 14

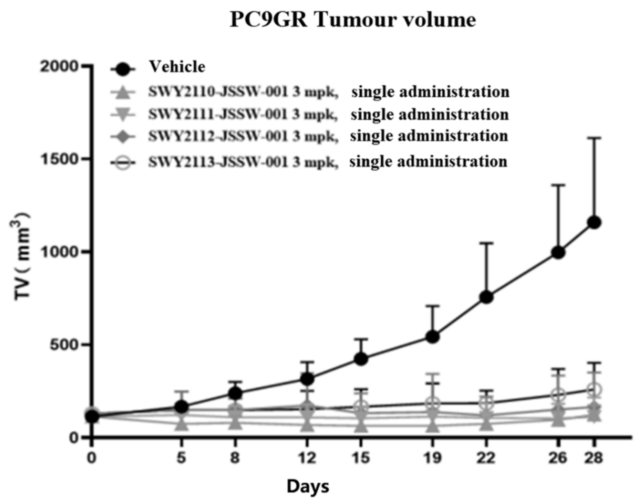


Figure 15

**INTERNATIONAL SEARCH REPORT**

International application No.
**PCT/CN2024/089266**

**A. CLASSIFICATION OF SUBJECT MATTER**

A61K47/68(2017.01)i; A61K31/4745(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, WPABS, WPABSC, STN on web, ISI web of science, Pubmed, Bing, NCBI, EBI, 中国专利生物序列检索数据库, China Patent Biological Sequence Search Database: SWY2110, SWY2111, SWY2112, SWY2113, SEQ ID NOs: 21-40, EGFR, HER, 石药集团巨石生物制药有限公司, 药物, 偶联, conjugate, 喜树碱, 依喜替康, exatecan, camptothecin, 连接子, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | CN 116135232 A (CSPC JUSHI BIOLOGICAL PHARMACEUTICAL CO., LTD.) 19 May 2023 (2023-05-19)<br>abstract, and description, paragraphs 0017-0091, amino acid sequences, table 30, and figures 21-24 | 1-13 |
| A | WO 2020063676 A1 (JIANGSU HENGRUI MEDICINE CO., LTD. et al.) 02 April 2020 (2020-04-02)<br>claims 38 and 45, and description, page 15, line 1, and page 23, last paragraph | 1-13 |
| A | WO 2016131409 A1 (SHANGHAI MIRACOGEN INC.) 25 August 2016 (2016-08-25)<br>description, page 19 | 1-13 |
| A | CN 106163559 A (DAIICHI SANKYO COMPANY, LIMITED et al.) 23 November 2016 (2016-11-23)<br>claims 1-38 | 1-13 |
| A | CN 108864290 A (SHANGHAI JMT BIO INC.) 23 November 2018 (2018-11-23)<br>claims 1-8, and description, amino acid sequence listing | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 August 2024** | **07 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/089266**

**C. DOCUMENTS CONSIDERED TO BE RELEVANT**

| Category* | Citation of document, with indication, where appropriate, of the relevant passages | Relevant to claim No. |
|---|---|---|
| A | CN 112566942 A (DAIICHI SANKYO COMPANY, LIMITED) 26 March 2021 (2021-03-26)<br>description, paragraphs 597-613 | 1-13 |
| A | CN 110835375 A (SHANGHAI NOVAMAB BIOPHARMACEUTICALS CO., LTD.) 25 February 2020 (2020-02-25)<br>abstract, and description, amino acid sequence listing | 1-13 |

Form PCT/ISA/210 (second sheet) (July 2022)

**INTERNATIONAL SEARCH REPORT**

International application No.
**PCT/CN2024/089266**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-13**
because they relate to subject matter not required to be searched by this Authority, namely:

Claims 9-13 relate to a method for treating a tumor, and the application relates to the use of administering a drug to a living human body for treating a tumor. Therefore, said claims do not comply with PCT Rule 39.1(iv). A search is carried out on the basis that claims 9-13 relate to the use in the preparation of a drug for treating a tumor.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/089266**

| Patent document cited in search report | | | Publication date (day/month/year) | Patent family member(s) | | | Publication date (day/month/year) |
|---|---|---|---|---|---|---|---|
| CN | 116135232 | A | 19 May 2023 | AU | 2022389555 | A1 | 04 July 2024 |
| | | | | CA | 3238578 | A1 | 25 May 2023 |
| | | | | WO | 2023088382 | A1 | 25 May 2023 |
| WO | 2020063676 | A1 | 02 April 2020 | ZA | 202102544 | B | 25 October 2023 |
| | | | | CA | 3114137 | A1 | 02 April 2020 |
| | | | | JP | 2022511351 | A | 31 January 2022 |
| | | | | BR | 112021004656 | A2 | 01 June 2021 |
| | | | | EP | 3858386 | A1 | 04 August 2021 |
| | | | | EP | 3858386 | A4 | 12 October 2022 |
| | | | | KR | 20210066833 | A | 07 June 2021 |
| | | | | MX | 2021003382 | A | 27 May 2021 |
| | | | | US | 2021353764 | A1 | 18 November 2021 |
| | | | | AU | 2019349207 | A1 | 08 April 2021 |
| | | | | TW | 202027795 | A | 01 August 2020 |
| | | | | TWI | 826543 | B | 21 December 2023 |
| WO | 2016131409 | A1 | 25 August 2016 | US | 2018036423 | A1 | 08 February 2018 |
| | | | | US | 10792370 | B2 | 06 October 2020 |
| | | | | CN | 111529717 | A | 14 August 2020 |
| CN | 106163559 | A | 23 November 2016 | KR | 20240008415 | A | 18 January 2024 |
| | | | | KR | 20200077620 | A | 30 June 2020 |
| | | | | KR | 102186027 | B1 | 03 December 2020 |
| | | | | MX | 2016010533 | A | 12 December 2016 |
| | | | | KR | 20220068270 | A | 25 May 2022 |
| | | | | KR | 102445502 | B1 | 21 September 2022 |
| | | | | KR | 20190004837 | A | 14 January 2019 |
| | | | | KR | 102127623 | B1 | 29 June 2020 |
| | | | | JP | 2022180416 | A | 06 December 2022 |
| | | | | JP | 7383772 | B2 | 20 November 2023 |
| | | | | JP | 6148422 | B1 | 14 June 2017 |
| | | | | JP | 2017197515 | A | 02 November 2017 |
| | | | | CA | 2939802 | A1 | 15 October 2015 |
| | | | | CA | 2939802 | C | 01 November 2022 |
| | | | | RS | 61711 | B1 | 31 May 2021 |
| | | | | US | 2019151328 | A1 | 23 May 2019 |
| | | | | US | 11298359 | B2 | 12 April 2022 |
| | | | | TW | 201542230 | A | 16 November 2015 |
| | | | | TWI | 704928 | B | 21 September 2020 |
| | | | | LT | 3129063 | T | 25 June 2021 |
| | | | | HRP | 20210593 | T1 | 14 May 2021 |
| | | | | RU | 2019143766 | A | 20 February 2020 |
| | | | | KR | 20200136052 | A | 04 December 2020 |
| | | | | KR | 102239413 | B1 | 12 April 2021 |
| | | | | JP | 2017503784 | A | 02 February 2017 |
| | | | | JP | 6105171 | B2 | 29 March 2017 |
| | | | | KR | 20210115056 | A | 24 September 2021 |
| | | | | KR | 102351755 | B1 | 14 January 2022 |
| | | | | JP | 2019135248 | A | 15 August 2019 |
| | | | | JP | 6707696 | B2 | 10 June 2020 |
| | | | | KR | 20230021173 | A | 13 February 2023 |
| | | | | KR | 102624244 | B1 | 11 January 2024 |

Form PCT/ISA/210 (patent family annex) (July 2022)

**INTERNATIONAL SEARCH REPORT**
**Information on patent family members**

International application No.
**PCT/CN2024/089266**

| Patent document cited in search report | Publication date (day/month/year) | Patent family member(s) | | | Publication date (day/month/year) |
|---|---|---|---|---|---|
| | | IL | 300540 | A | 01 April 2023 |
| | | IL | 300540 | B1 | 01 April 2024 |
| | | IL | 248239 | A0 | 30 November 2016 |
| | | IL | 248239 | B | 01 July 2022 |
| | | SI | 3129063 | T1 | 31 August 2021 |
| | | TW | 202108176 | A | 01 March 2021 |
| | | TWI | 745021 | B | 01 November 2021 |
| | | US | 2017021031 | A1 | 26 January 2017 |
| | | US | 10383878 | B2 | 20 August 2019 |
| | | ES | 2859648 | T3 | 04 October 2021 |
| | | AU | 2020200227 | A1 | 06 February 2020 |
| | | AU | 2020200227 | B2 | 07 October 2021 |
| | | JP | 2021169515 | A | 28 October 2021 |
| | | JP | 7138750 | B2 | 16 September 2022 |
| | | KR | 20210041126 | A | 14 April 2021 |
| | | NZ | 722668 | A | 23 February 2024 |
| | | IL | 311108 | A | 01 April 2024 |
| | | BR | 112016017893 | A2 | 17 October 2017 |
| | | BR | 112016017893 | B1 | 23 August 2022 |
| | | CY | 1124071 | T1 | 27 May 2022 |
| | | SG | 10201907807 | XA | 27 September 2019 |
| | | RU | 2016143351 | A | 15 May 2018 |
| | | RU | 2016143351 | A3 | 05 March 2019 |
| | | RU | 2711640 | C2 | 17 January 2020 |
| | | IL | 293177 | A | 01 July 2022 |
| | | IL | 293177 | B1 | 01 March 2023 |
| | | IL | 293177 | B2 | 01 July 2023 |
| | | EP | 3129063 | A1 | 15 February 2017 |
| | | EP | 3129063 | B1 | 27 January 2021 |
| | | DK | 3129063 | T3 | 06 April 2021 |
| | | PL | 3129063 | T3 | 12 July 2021 |
| | | JP | 2020143114 | A | 10 September 2020 |
| | | JP | 6918182 | B2 | 11 August 2021 |
| | | SG | 11201608309 | PA | 29 November 2016 |
| | | EP | 3789042 | A1 | 10 March 2021 |
| | | KR | 20160144396 | A | 16 December 2016 |
| | | KR | 101937549 | B1 | 10 January 2019 |
| | | JP | 2024019172 | A | 08 February 2024 |
| | | KR | 20220012402 | A | 03 February 2022 |
| | | KR | 102399277 | B1 | 18 May 2022 |
| | | HUE | 054411 | T2 | 28 September 2021 |
| | | MX | 2021004884 | A | 15 June 2021 |
| | | AU | 2021286320 | A1 | 20 January 2022 |
| | | AU | 2021286320 | B2 | 18 April 2024 |
| | | AU | 2015245122 | A1 | 18 August 2016 |
| | | AU | 2015245122 | B2 | 24 October 2019 |
| | | TW | 202218685 | A | 16 May 2022 |
| | | TWI | 826828 | B | 21 December 2023 |
| | | PH | 12016501711 | A1 | 19 December 2016 |
| | | KR | 20220132025 | A | 29 September 2022 |

Form PCT/ISA/210 (patent family annex) (July 2022)

**INTERNATIONAL SEARCH REPORT**
**Information on patent family members**

International application No.
**PCT/CN2024/089266**

| Patent document cited in search report | | | Publication date (day/month/year) | Patent family member(s) | | | Publication date (day/month/year) |
|---|---|---|---|---|---|---|---|
| | | | | KR | 102495426 | B1 | 06 February 2023 |
| | | | | WO | 2015155998 | A1 | 15 October 2015 |
| | | | | JP | 2017222638 | A | 21 December 2017 |
| | | | | JP | 6513128 | B2 | 15 May 2019 |
| | | | | MY | 195180 | A | 11 January 2023 |
| | | | | PT | 3129063 | T | 01 April 2021 |
| CN | 108864290 | A | 23 November 2018 | US | 2020157223 | A1 | 21 May 2020 |
| | | | | US | 11518810 | B2 | 06 December 2022 |
| | | | | EP | 3623388 | A1 | 18 March 2020 |
| | | | | EP | 3623388 | A4 | 04 August 2021 |
| | | | | US | 2023082273 | A1 | 16 March 2023 |
| | | | | TW | 201843181 | A | 16 December 2018 |
| | | | | TWI | 816673 | B | 01 October 2023 |
| | | | | KR | 20200003845 | A | 10 January 2020 |
| | | | | AU | 2018264321 | A1 | 12 December 2019 |
| | | | | RU | 2019138624 | A | 09 June 2021 |
| | | | | RU | 2019138624 | A3 | 22 July 2021 |
| | | | | WO | 2018205936 | A1 | 15 November 2018 |
| | | | | JP | 2020520249 | A | 09 July 2020 |
| | | | | JP | 7296367 | B2 | 22 June 2023 |
| CN | 112566942 | A | 26 March 2021 | AU | 2019311557 | A1 | 04 February 2021 |
| | | | | CA | 3107417 | A1 | 30 January 2020 |
| | | | | CA | 3107417 | C | 17 October 2023 |
| | | | | KR | 20210038904 | A | 08 April 2021 |
| | | | | IL | 280295 | A | 25 March 2021 |
| | | | | JP | 2024050683 | A | 10 April 2024 |
| | | | | TW | 202019972 | A | 01 June 2020 |
| | | | | SG | 11202100653 | YA | 25 February 2021 |
| | | | | US | 2021283269 | A1 | 16 September 2021 |
| | | | | JPWO | 2020022363 | A1 | 02 August 2021 |
| | | | | EP | 3828206 | A1 | 02 June 2021 |
| | | | | EP | 3828206 | A4 | 20 April 2022 |
| | | | | BR | 112021001194 | A2 | 27 April 2021 |
| | | | | WO | 2020022363 | A1 | 30 January 2020 |
| CN | 110835375 | A | 25 February 2020 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211461614
- US 4816567 A


### Non-patent literature cited in the description


- **KOHLER et al.** *Nature*, 1975